# EUROPEAN PATENT APPLICATION

(11) **EP 1 958 941 A1**
(43) Date of publication of application: **20.08.2008**
(21) Application number: 07405045.1
(22) Date of filing: 14.02.2007
(51) Int. Cl.: C07D 263/20, C07D 263/24, C07D 413/06, C07D 413/12, A61K 31/421, A61K 31/422, A61K 31/4439, A61P 9/10, A61P 3/00

(54) **Oxazolidinones and their use as hypocholesterolemic agents**

(71) Applicant: Lipideon Biotechnology AG, 8008 Zürich (CH)
(72) Inventor: Hauser, Helmut, 8008 Zürich (CH); Carreira, Erick, 8126 Zumikon (CH)
(74) Representative: Carreira-Staubli, Andrea

(57) **Abstract**

The present invention relates to novel hypocholesterolemic compounds of formula I 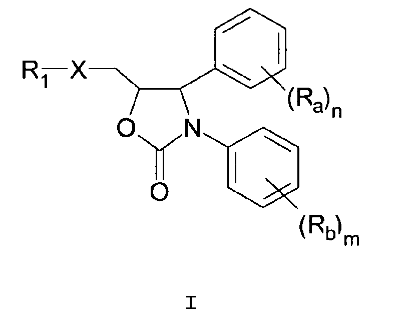 useful in the treatment and prevention of atherosclerosis and for the reduction of cholesterol levels as well as to pharmaceutical compositions comprising said compounds alone or in combination with other active agents.

## Description

### Field of the Invention

The present invention relates to novel hypocholesterolemic compounds useful in the treatment and prevention of atherosclerosis and for the reduction of cholesterol levels as well as to pharmaceutical compositions comprising said compounds alone or in combination with other active agents.

### Background

Atherosclerotic coronary heart disease represents the major cause for death and cardiovascular morbidity in the western world. Risk factors for atherosclerotic coronary heart disease include hypertension, diabetes mellitus, family history, male gender, cigarette smoke as well as serum cholesterol. Elevated concentrations of serum cholesterol have been demonstrated by a number of clinical studies to be a major contributing factor in the development and progression of atherosclerosis, which is characterized by the formation of cholesterol-containing plaques in the aorta and lesser arteries.

In mammals, 1/3 of the serum cholesterol is derived from exogenous dietary sources which enters the body through absorption in the intestine and 2/3 of the serum cholesterol are derived through endogenous de novo synthesis in the liver involving a complex set of enzyme-catalyzed reactions and regulatory mechanisms.

It has been reported in the literature that intestinal cholesterol absorption is an energy-independent, protein-mediated process (Hauser, H. et al, Biochemistry 1998, 37, 17843-17850; Schulthess, G. et al, Biochemistry 2000, 39, 12623-12631; Werder, M. et al, Biochemistry 2001, 40, 11643-11650) rather than a passive diffusion process. The proteins facilitating intestinal cholesterol absorption were identified as two brush border membrane-resident scavenger receptors (Hauser, H. et al, Biochemistry 1998, 37, 17843-17850; Werder, M. et al, Biochemistry 2001, 40, 11643-11650). Both in vitro and *in vivo* animal experiments confirmed the presence of these two scavenger receptors in the intestinal BBM and proved that they are partially responsible for the protein-mediated cholesterol uptake (van Bennekum, A. et al Biochemistry 2005, 44, 4517-4525).

In view of the above, research efforts have focused on identifying classes of compounds that are able to interfere with the above-described processes. Literature reports have shown that compound classes having either a 2-azetidione- or an oxazolidinone-scaffold may be useful in lowering cholesterol and/or in inhibiting the formation of cholesterol-containing lesions in mammalian arterial walls:

The former compound class having a 2-azetidione-scaffold is for example disclosed in WO 93/02048, WO 94/17038, WO 95/08532, WO 95/26334, U.S. 5,633,246, which describe 2-azetidinone compounds with different substituents at the 3-position, and U.S. 5,756,470 which discloses 2-azetidinones having varying sugar-substituents at the 4-position. Extensive studies on the 2-azetidinones have shown that their activity was dependent on the nature and configuration of the various sidechains. For example it has been reported that their activity was dependent on the presence of a C3-arylalkyl sidechain having a stereogenic center and an alkylchainlength of preferably exactly 3 carbon atoms (J. Med. Chem., 1998, 41, 973; J. Med. Chem., 1996, 39, 3684). These findings resulted in the development of the most prominent representative of the 2-azetidinones, Ezetimibe (also known under trade names Zetia^{™} and Ezetrol^{®}), which is in use as a cholesterol-lowering drug in monotherapy and in dual therapy combined with a statin. However, there are still general drawbacks associated with the 2-azetidinones, which may include absorption and metabolization upon administration into the pharmalogically active glucuronide (van Heek, M. et al. Br. J. Pharmacol. 2000, 129, 1748-1754) and side effects such as allergic reactions, e.g. rash and angiodema, which may result from the hydrolytic instability of the 2-azetidinone ring scaffold.

The latter compound class having an oxazolidinone scaffold has recently been identified to be a promising candidate for inhibition of the above-described processes of cholesterol absorption by presenting an optimal arrangement of suitable substituents at the C4-, C5-, and N3-position, respectively (WO 2005/033100).

Applicants have now found that the high pharmacological activity, i.e. inhibitory activity, of oxazolidinones could be considerably altered upon specific modification of the C5-substituent.

As indicated hereinabove, prior art in this field has reported that activity of the well studied azetidinones depended on the nature of the C3-side chain, leading to a highly active compound having a sterogenic center and an optimal C₃ chain length.

In contrast, applicants have discovered that such a correlation does not apply to compounds with an oxazolidinone scaffold. In fact, applicants have unexpectedly observed improved pharmacological activity for compounds with an oxazolidinone scaffold having an acceptor/donor site (presumably for interaction with the biologicl target site) linked in close proximity to the C5 ring atom. In particular, improved activity was found for oxazolidinone compounds having an achiral substituent at the C5-ringatom comprising an electronegative group, such as -O-, -NH-, -N=, -NH-CO-, -NH-CO-O-, -NH-CO-NH-, or combinations thereof. In contrast to prior art, said electronegative group need simply be linked through a methylenebridge to the C5-ringatom.

It was further found that said electronegative group, which hereinafter may also be refered to as heteroatom linker, could also be in a cyclic from, i.e. as a heteroaryl group. Thus surprisingly, a steric restriction of the acceptor/donor site by reducing the chain length and possibly fixing the electronegative group in a cyclic form, seemed to be favourable.

Thus as opposed to the state of the art, truncation of the alkylchain length to one carbon atom only and deletion of the sterogenic center of the sidechain in question, resulted in improved activity in the case of oxazolidinones according to the present invention.

Moreover, applicants were able to devise synthetic procedures allowing to generate libraries of compounds of the invention having an achiral C5-sidechain comprising a heteroatom linker attached to C5 via a methylenebridge (see also schemes 1 and 2).

Thus, the compounds of the present invention with the structural characteristics as depicted hereinafter are able to inhibit the protein-mediated process mentioned above by which cholesterol absorption is mediated, while overcoming the above described disadvantages of compounds known in the art.

### Summary of the Invention

The present invention relates to novel hypocholesterolemic compounds useful in the treatment and prevention of atherosclerosis and for the reduction of cholesterol levels as well as to pharmaceutical compositions comprising said compounds alone or in combination with other active agents.

In particular, the present invention relates to oxazolidinone compounds with an achiral C5-sidechain having as key structural element a methylene-linked electronegative group. This methylene-linked electronegative group (also referred to as hetereoatom linker) includes groups such as -O-, -NH-, -N=, -NH-CO-, - NH-CO-O-, -NH-CO-NH-, or combinations thereof, which are in acyclic or cyclic form, i.e. forming a heteroaryl group.

Thus in a first aspect the present invention relates to compounds of formula I, or a pharmaceutically acceptable salt or solvate thereof, 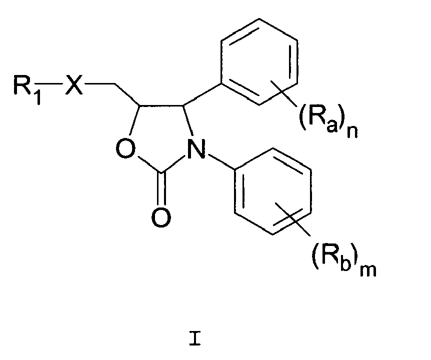 wherein
- X: represents a heteroatom linker selected from -O-, -NH-, - N=, -NH-CO-, -O-CO-NH-, -NH-CO-NH-, a heteroaryl group, or combinations thereof;
- R₁: represents H, or R₂-substituted aryl Ar, wherein R₂ represents H, (C1-10) alkyl, -OR₃, -OCOR₃, -NR₃R₄, - NR₃(CO)R₄, -COOR₃, -CONR₃R₄, -CH=CHCOOR₃, -CF₃, -CN, -NO₂, or halogen, wherein R₃ and R₄ independently of each other represent H, (C1-10)alkyl, aryl or heteroaryl;
- Rₐ: represents independently of each other H, (C1-10)alkyl, - OR₅, -O(CO)R₅, -NR₅R₆, -NR₅(CO)R₆, halogen, -NO₂, or a polar moiety, wherein R₅ and R₆ represent independently of each other H, (C1-10)alkyl, aryl or heteroaryl;
- R_{b}: represents independently of each other H, (C1-10)alkyl, - OR₇, -O(CO)R₇, -NR₇R₈, -NR₇(CO)R₈, -COOR₇, -CONR₇R₈, - CH=CHCOOR₇, halogen, -CF₃, -CN, -NO₂, or a polar moiety, wherein R₇ and R₈ represent independently of each other H, (C1-10)alkyl, aryl or heteroaryl, and
- n, m: are independently of each other 0, 1 or 2.

In one embodiment the heteroaryl group is a five- or six-membered ring having 1 to 3 heteroatoms selected from N, O, S, and benzo-fused derivatives thereof. Preferred examples include but are not limited to, pyridine, pyrimidine, oxazole, thiazole, imidazole, triazole, pyrazole, and phthalimide.

In another embodiment R₂-substituted aryl Ar is a five- to ten-membered, preferably five- or six-membered aromatic, carbocyclic or heterocyclic group. Preferred examples include but are not limited to, phenyl, pyridyl, naphthyl, wherein R₂ is selected from H, (C1-10)alkyl, -OR₃, -NR₃R₄, -COOR₃, -CONR₃R₄, -CF₃, or halogen, wherein R₃ and R₄ preferably represent H or (C1-10)alkyl.

In a further embodiment, the polar moiety is selected from glucoronides, polyhydroxy groups, 2,3-dihydroxy propoxy groups, tartrate groups, N-(2-Hydroxyethyl)-iminodiacetic acid groups, tetrazolium salt groups, phosphate groups, sulfonate groups, sulfonic acid groups, aminoalkyl groups, alkylammonium groups, quaternary ammonium salts and derivatives thereof.

In yet a further embodiment, n is 1 or m is 1, preferably both n and m are 1.

In a further embodiment the substituents in the 4- and 5- position are in trans configuration to each other

In another aspect, the present invention relates to a pharmaceutical composition comprising a therapeutically effective amount of a compound of formula I with a pharmaceutically acceptable carrier.

In another aspect, the present invention relates to the use of of a compound of formula I or a pharmaceutical composition thereof for the treatment or prevention of artheriosclerosis or for the reduction of cholesterol levels, optionally in combination with one or more other active agents.

In yet another aspect, the present invention relates to a kit comprising a pharmaceutical composition of the invention

### Detailed Description of the Invention

The present invention relates to novel hypocholesterolemic compounds useful in the treatment and prevention of atherosclerosis and for the reduction of cholesterol levels as well as to pharmaceutical compositions comprising said compounds alone or in combination with other active agents.

In particular, the present invention relates to oxazolidinone compounds with an achiral C5-sidechain having as key structural element a methylene-linked electronegative group. This methylene-linked electronegative group (also referred to as hetereoatom linker) includes groups such as -O-, -NH-, -N=, -NH-CO-, - NH-CO-O-, -NH-CO-NH-, or combinations thereof, which are in acyclic or cyclic form, i.e. forming a heteroaryl group.

More specifically, the present invention relates to a compound according to formula I or a pharmaceutically acceptable salt or solvate thereof, 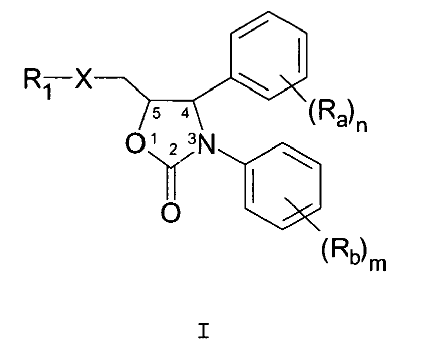 wherein
- X: represents a heteroatom linker selected from -O-, -NH-, - N=, -NH-CO-, -O-CO-NH-, -NH-CO-NH-, a heteroaryl group, or combinations thereof;
- R₁: represents H, or R₂-substituted aryl Ar, wherein R₂ represents H, (C1-10)alkyl, -OR₃, -OCOR₃, -NR₃R₄, - NR₃(CO)R₄, -COOR₃, -CONR₃R₄, -CH=CHCOOR₃, -CF₃, -CN, -NO₂, or halogen, wherein R₃ and R₄ represent independently of each other H, (C1-10)alkyl, aryl or heteroaryl;
- Rₐ: represents independently of each other H, (C1-10)alkyl, - OR₅, -O(CO)R₅, -NR₅R₆, -NR₅(CO)R₆, halogen, -NO₂, or a polar moiety, wherein R₅ and R₆ represent independently of each other H, (C1-10)alkyl, aryl or heteroaryl,
- R_{b}: represents independently of each other H, (C1-10)alkyl, - OR₇, -O(CO)R₇, -NR₇R₈, -NR₇(CO)R₈, -COOR₇, -CONR₇R₈, - CH=CHCOOR₅, halogen, -CF₃, -CN, -NO₂, or a polar moiety, wherein R₇ and R₈ represent independently of each other H, (C1-10)alkyl, aryl or heteroaryl and
- n, m: are independently of each other 0, 1 or 2.

In one preferred embodiment X is selected from -O-, -NH-, -N=, - NH-CO-, -O-CO-NH-, -NH-CO-NH-, or combinations thereof. Preferably, when (Rₐ)ₙ equals -OBn in para position and (R_{b})ₘ equals -F in para position, -X-R₁ may not be -OH.

In another preferred embodiment X is a heteroaryl group.

The term "heteroaryl group" or "heteroaryl" should be understood to include an aromatic ring system of 5 to 10, preferably 5, 6 or 10, more preferably 5 or 6 ring atoms, in which one or more of the atoms in the ring system is/are atoms other than carbon, for example nitrogen, oxygen or sulfur. Preferably, the heteroaryl is a 5- or 6-membered aromatic ring having 1 to 3 heteroatoms selected from N, O, S, preferably N and O, and benzofused derivatives thereof. Examples of suitable 6-membered heteroaryl groups include pyridine, pyrimidine, pyrazine, pyridazine and the like. Examples of useful 5-membered heteroaryl rings include furan, pyrrole, triazole, thiazole, isothiazole, imidazole, pyrazole, oxazole and isoxazole. Useful bicyclic groups are benzo-fused ring systems derived from the heteroaryl groups named above, e.g., quinoline, phthalazine, quinazoline, benzofuran, phthaleimide and indole. Most preferred examples are pyridine, pyrimidine, oxazole, thiazole, imidazole, triazole, pyrazole, and phthaleimide.

R₂ should be understood to represent one or more substituents, preferably one substituent, independently selected from H, (C1-10)alkyl, -OR₃, -OCOR₃, -COOR₃, -CONR₃R₄, -CH=CHCOOR₃, -CF₃, -CN, - NO₂, or halogen, wherein R₃ and R₄ represent H or (C1-10) alkyl. Preferably R₂ represents H, -OH, -O(C1-10)alkyl, -CF₃, -CN, or halogen, most preferably H, -O(C1-10)alkyl, or halogen.

The term "aryl Ar" should be understood to include a monocyclic, bicyclic, carbocyclic or heterocyclic ring system having 5 to 10, preferably 5, 6 or 10, more preferably 5 or 6 ring atoms. If Ar is a carbocyclic ring system, non-limiting examples of suitable aryl groups include phenyl, (1- or 2-)naphthyl or tetraline groups, most preferably phenyl groups. If Ar is a heterocyclic ring system, it is as defined hereinbefore under heteroaryl group. The aryl Ar can be substituted with one or more substituents R₂, which may be the same or different, and are selected from a group as defined hereinabove. The substituents may be in any position. For example in monosubstituted phenyl residues the substituent can be located in the ortho-position, the meta-position or the para-position, preferably in the para-position. If phenyl is substituted twice, the substituents can be in the ortho,meta-position (all possibilities), the ortho,para-position, the ortho,ortho-position, the meta,meta-position, or the meta,para-position. In a preferred embodiment, Ar is a five- or six-membered aromatic, cyclic or heterocyclic group, more preferably phenyl, pyridyl, naphthyl.

The term "polar moiety" should be understood to include any polar moiety which enhances and thus imparts impermeability to the compounds of the invention. This includes uncharged and charged (both positively and negatively) groups. Typical examples of such moieties include glucoronides, polyhydroxy groups, 2,3-dihydroxy propoxy groups, tartrate groups, N-(2-Hydroxyethyl)-iminodiacetic acid groups, tetrazolium salt groups, phosphate groups, sulfonate groups, sulfonic acid groups such as -OSO₂Me, - OSO₂W wherein W represents Phenyl (Ph) or isomers of salicylic acid (all combinations of disubstituted phenyl with OH and COOH substituents), aminoalkyl groups, alkylammonium groups, quaternary ammonium salts and derivatives thereof. Preferred groups include polyhydroxy groups, tartrate groups, N-(2-Hydroxyethyl)-iminodiacetic acid groups, phosphate groups, sulfonate groups, aminoalkyl groups, alkylammonium groups, and derivatives thereof, more preferably aminoalkyl groups, alkylammonium groups, and derivatives thereof.

Rₐ preferably represents independently of each other H, (C1-10) alkyl, -OR₅, halogen, -NO₂, or a polar moiety, wherein R₅ represents H, (C1-10)alkyl, aryl or heteroaryl; preferably H, (C1-10)alkyl, -Phe or -Bn; more preferably H, -OR₅, halogen, aminoalkyl groups or alkylammonium groups, wherein R₅ represents H or -Bn, most preferably H, -OH, or -OBn.

R_{b} preferably represents independently of each other H, (C1-10)alkyl, -OR₇, -O(CO)R₇, -NR₇R₈, -NR₇(CO)R₈, -COOR₇, -CONR₇R₈, - CH=CHCOOR₇, halogen, -CF₃, -CN, -NO₂, or a polar moiety, wherein R₇ and R₈ represent H, (C1-10)alkyl, aryl or heteroaryl; preferably H, (C1-10)alkyl, -Phe or -Bn; more preferably H, (C1-10)alkyl, -OR₇, -COOR₇, -CONR₇R₈, halogen, -CF₃, or a polar moiety, such as aminoalkyl groups or alkylammonium groups, wherein R₇ and R₈ represent H or (C1-10)alkyl.

In one embodiment n is 1. In another embodiment m is 1. In yet a further embodiment both n and m are 1.

The term "alkyl" should be understood to include straight chain and branched hydrocarbon groups having from 1 to 10, preferably 1 to 6, more preferably from 1 to 3 carbon atoms, which may be optionally substituted. Non-limiting examples of suitable lower alkyl groups include methyl, ethyl, n-propyl, isopropyl, n-butyl, t-butyl, n-pentyl, fluoromethyl and trifluoromethyl.

The term "branched" should be understood to represent a linear straight chain hydrocarbon group having one or more lower alkyl groups such as methyl, ethyl or propyl, attached to it.

The term "halogen" (or "hal") should be understood to include fluoro, chloro, bromo, iodo, preferably, fluoro and chloro, most preferably, fluoro.

The designation -Phe stands for phenyl and the designation -Bn stands for benzyl.

It is understood that all isomers, including enantiomers, stereoisomers, rotamers, tautomers and racemates of the compounds of formula I are contemplated as being part of this invention. The invention includes stereoisomers in optically pure form and in admixture, including racemic mixtures. Isomers can be prepared using conventional techniques, either by reacting optically pure or optically enriched starting materials or by separating isomers of a compound of formula I. In a preferred embodiment the stereochemistry in the central ring is such that the substituents at the 4- and 5-position are in trans configuration to each other.

In a specific embodiment the present invention relates to compounds of the present invention having formula II or a pharmaceutically acceptable salt or solvate thereof, 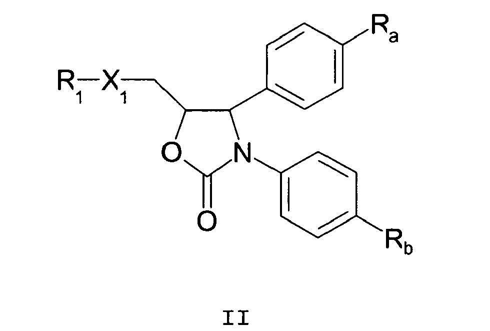 wherein
- X₁: represents an heteroatom linker selected from -O-, -NH-, -N=, -NH-CO-, -O-CO-NH-, -NH-CO-NH-, or combinations thereof,
- R₁: represents H or R₂-substituted aryl Ar, wherein R₂ represents H, -OH, -O(C1-10)alkyl, -CF₃, -CN, or halogen;
- Rₐ: represents H, (C1-10)alkyl, -OR₅, halogen, -NO₂, or a polar moiety, wherein R₅ represents H, (C1-10)alkyl, -Phe, or -Bn; and
- R_{b}: represents H, (C1-10)alkyl, -OR₇, -COOR₇, -CONR₇R₈, halogen, -CF₃, or a polar moiety, wherein R₇ and R₈ represent H or (C1-10)alkyl.

Preferably, when Rₐ and R_{b} represent -OBn and -F, respectively - X₁-R₁ may not be -OH.

Preferably Ar is selected from five- to ten-membered rings, preferably five- or six-membered rings, preferably phenyl, pyridyl, naphthyl.

In a further specific embodiment the present invention relates to compounds of the present invention having formula III or a pharmaceutically acceptable salt or solvate thereof, 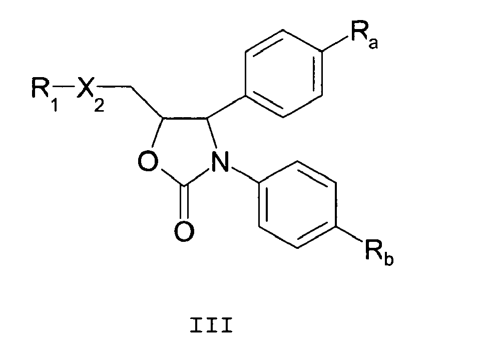 wherein
- X₂: represents a five- or six-membered ring having 1 to 3 heteroatoms selected from N, O, S and benzo-fused derivatives thereof;
- R₁: represents H or R₂-substituted aryl Ar, wherein R₂ represents H, -OH, -O(C1-10)alkyl, -CF₃, -CN, or halogen;
- Rₐ: represents H, (C1-10)alkyl, -OR₅, halogen, -NO₂, or a polar moiety, wherein R₅ represents H, (C1-10)alkyl, -Phe, or -Bn; and
- R_{b}: represents H, (C1-10)alkyl, -OR₇, -COOR₇, -CONR₇R₈, halogen, -CF₃, or a polar moiety, wherein R₇ and R₈ represent H or (C1-10)alkyl.

Preferably, X₂ is selected from five- or six-membered rings having 1 to 3 heteroatoms selected from N, O, S and benzo-fused derivatives thereof, more preferably X₂ is selected from pyridine, pyrimidine, oxazole, thiazole, imidazole, triazole, pyrazole and phtaleimide.

Preferably Ar is selected from five- to ten-membered rings, preferably five- or six-membered rings, preferably phenyl, pyridyl, naphthyl.

More specifically, preferred compounds of the invention include for example compounds having formula IV 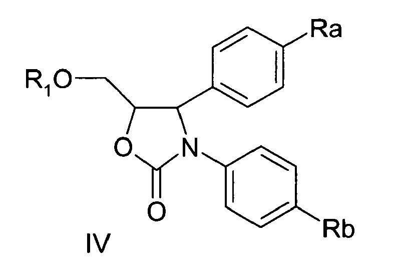 wherein
- R₁: represents H or R₂-substituted aryl Ar, wherein R₂ represents H, -OH, -O(C1-10)alkyl, -CF₃, -CN, or halogen;
- Rₐ: represents H, (C1-10) alkyl, -OR₅, halogen, -NO₂, or a polar moiety, wherein R₅ represents H, (C1-10)alkyl, -Phe, or -Bn; and
- R_{b}: represents H, (C1-10) alkyl, -OR₇, -COOR₇, -CONR₇R₈, halogen, -CF₃, or a polar moiety, wherein R₇ and R₈ represent H or (C1-10)alkyl.
with the proviso that, when Rₐ represents -OBn and R_{b} represents -F, R₁ may not be H.

Preferably Ar is selected from five- to ten-membered rings, preferably five- or six-membered rings, preferably phenyl, pyridyl, naphthyl.

Other preferred compounds of the invention include for example compounds having formulas V, VI or VII 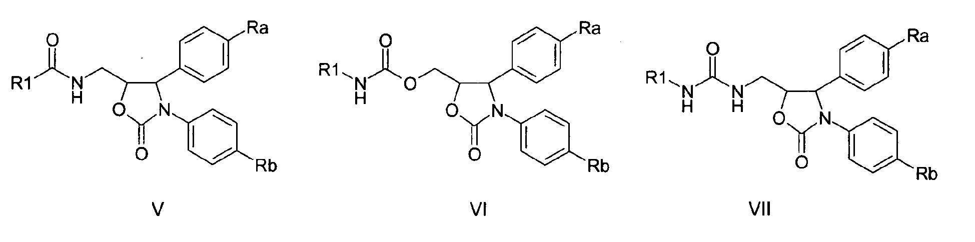 wherein
- R₁: represents H or R₂-substituted aryl Ar, wherein R₂ represents H, -OH, -O(C1-10)alkyl, -CF₃, -CN, or halogen;
- Rₐ: represents H, (C1-10) alkyl, -OR₅, halogen, -NO₂, or a polar moiety, wherein R₅ represents H, (C1-10)alkyl, -Phe, or -Bn; and
- R_{b}: represents H, (C1-10) alkyl, -OR₇, -COOR₇, -CONR₇R₈, halogen, -CF₃, or a polar moiety, wherein R₇ and R₈ represent H or (C1-10)alkyl.

Preferably Ar is selected from five- to ten-membered rings, preferably five- or six-membered rings, preferably phenyl, pyridyl, naphthyl.

Other preferred compounds of the invention wherein the heterolinker comprises a N- or C-linked diazole or triazole include for example compounds having formulas VIII, IX or X 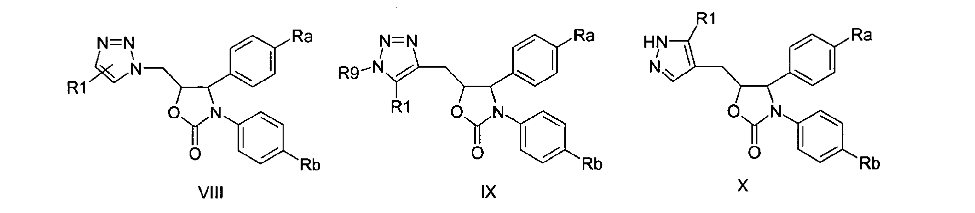 wherein
- R₁: represents H or R₂-substituted aryl Ar, wherein R₂ represents H, -OH, -O(C1-10)alkyl, -CF₃, -CN, or halogen;
- R₉: R₉ represents H, (C1-10)alkyl, -Phe, or -Bn;
- Rₐ: represents H, (C1-10)alkyl, -OR₅, halogen, -NO₂, or a polar moiety, wherein R₅ represents H, (C1-10)alkyl, -Phe, or -Bn; and
- R_{b}: represents H, (C1-10) alkyl, -OR₇, -COOR₇, -CONR₇R₈, halogen, -CF₃, or a polar moiety, wherein R₇ and R₈ represent H or (C1-10)alkyl.

Preferably Ar is selected from five- to ten-membered rings, preferably five- or six-membered rings, preferably phenyl, pyridyl, naphthyl.

Further preferred compounds of the invention wherein the heterolinker comprises a benzofused heteroaryl include for example compounds having formula XI 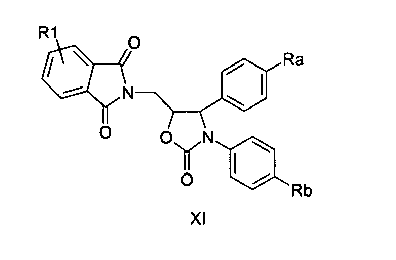 wherein
- R₁: represents H or R₂-substituted aryl Ar, wherein R₂ represents H, -OH, -O(C1-10)alkyl, -CF₃, -CN, or halogen;
- Rₐ: represents H, (C1-10) alkyl, -OR₅, halogen, -NO₂, or a polar moiety, wherein R₅ represents H, (C1-10)alkyl, -Phe, or -Bn; and
- R_{b}: represents H, (C1-10)alkyl, -OR₇, -COOR₇, -CONR₇R₈, halogen, -CF₃, or a polar moiety, wherein R₇ and R₈ represent H or (C1-10)alkyl.

Preferably Ar is selected from five- to ten-membered rings, preferably five- or six-membered rings, preferably phenyl, pyridyl, naphthyl.

The compounds of the invention may be prepared using methods of preparation known in the art (Kvaerno, L. et al Angew. Chem. Int. Ed. 2004, 43, 4653-4656

More conveniently, a modular synthetic route for easy access to a structurally diverse library of compounds has been developed (as indicated in schemes 1a, 1b and 2). This synthetic route uses as a central intermediate a hydroxymethyl- (or carboxy-) oxazolidinone compound, which can be obtained according to the following scheme 3. Thus, this modular synthetic route allows access to compounds of the invention having various heterolinkers at the C5-ring atom as well as great variablitiy at the C4- and N3-positions:

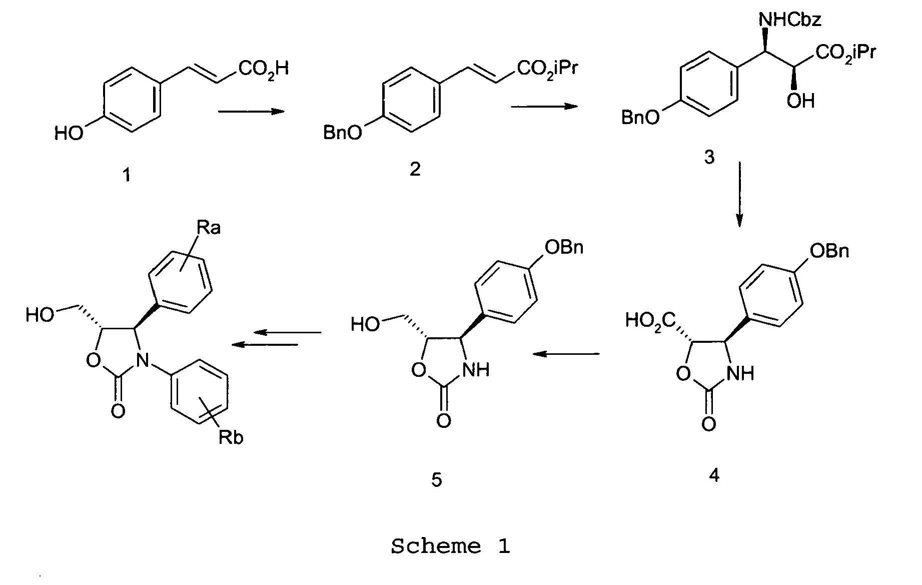

Thus, compounds of formulas V to VIII can for example be derived from compound of formula IV optionally via the azide intermediate of formula XIII according to scheme 2:

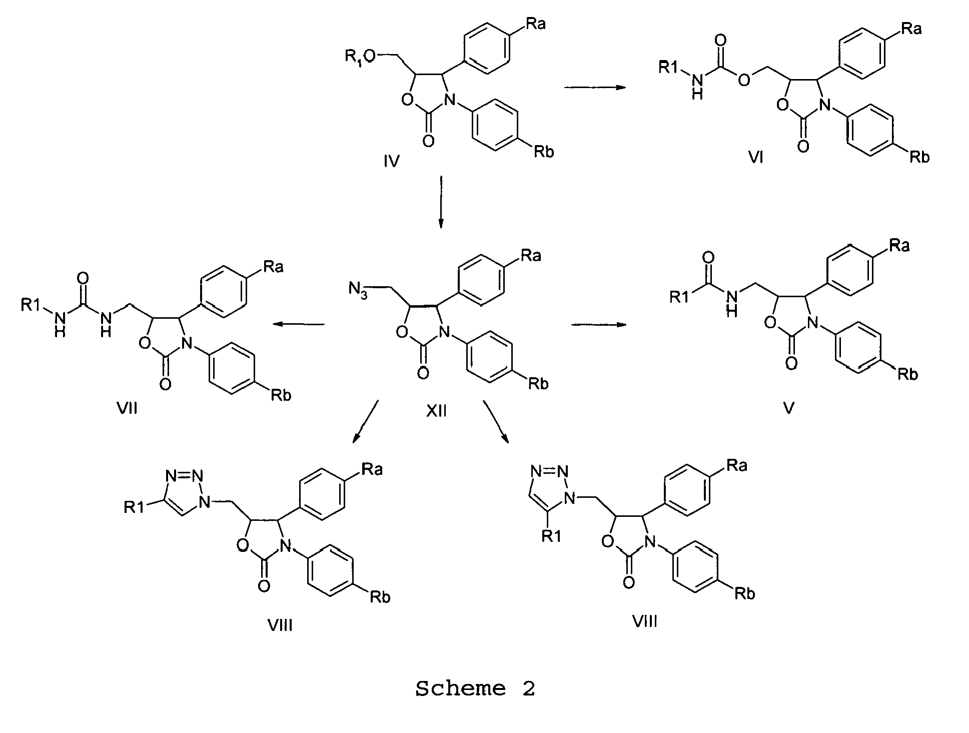

Compounds of formulas IX, X and XI can for example be derived from the carboxy intermediate of formula XIII according to scheme 3:

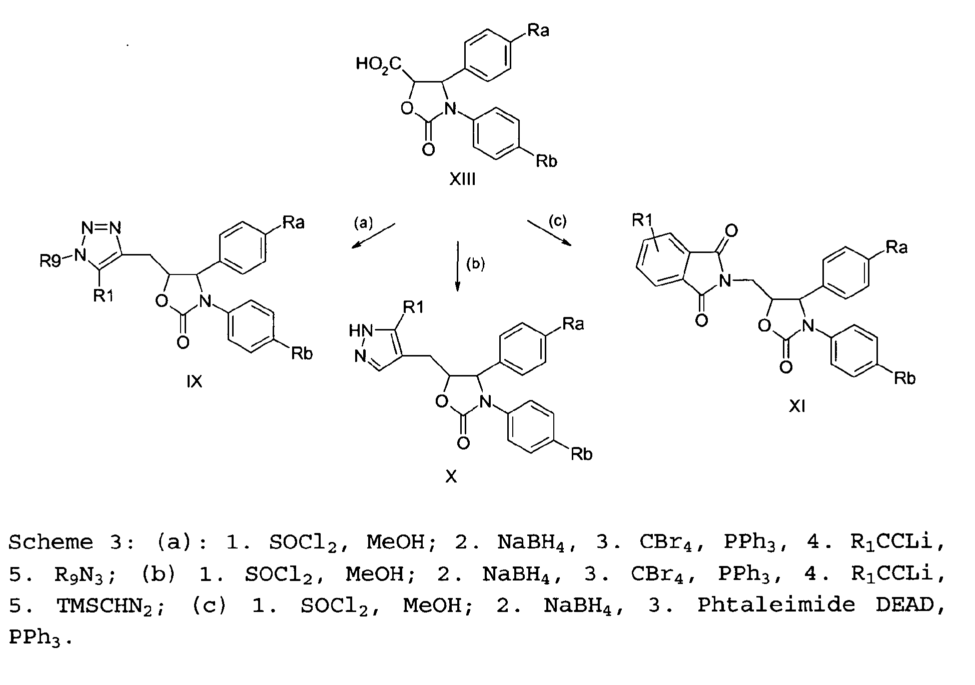

It has further been shown that the compounds of the invention display superior pharmacological activities and are able to overcome the drawbacks of known cholesterol-lowering agents using well-established methods in the art, e.g. evaluation of their inhibition of cholesterol uptake in rabbit brush border membrane vesicles (BBMV), in Caco-2 cells, and in mice (Hauser, H. et al, Biochemistry 1998, 37, 17843-17850; Schulthess, G. et al, Biochemistry 2000, 39, 12623-12631; Werder, M. et al, Biochemistry 2001, 40, 11643-11650; Boffelli, D. et al. FEBS Lett. 1997, 411, 7-1, Kvaerno, L. et. al. Angew. Chem. Int. Ed. 2004, 43, 4653-4656; Kvaerno, L. et. al. Org. Lett. 2005, 7, 1145-1148, van Bennekum, A. et. al. Biochemistry 2005, 44, 4517-4525).

Thus, the compounds of the invention and their pharmaceutically acceptable acid addition salts, exhibit pharmacological activity and are, therefore, useful as pharmaceuticals. The compounds of the invention have been shown to effectively inhibit cholesterol absorption and are therefore useful in the treatment and/or prevention of atherosclerosis and of the reduction of cholesterol levels.

Thus in yet a further aspect, the present invention is directed to a method of treatment and/or prevention of atherosclerosis, of the reduction of cholesterol levels and of treating or preventing a vascular condition, comprising administering to a mammal in need of such treatment an effective amount of a compound of the present invention.

The novel compounds of the present invention can be used, for example, in the form of pharmaceutical compositions containing a therapeutically effective amount of the active ingredient, if appropriate together with inorganic or organic, solid or liquid, pharmaceutically acceptable carriers suitable for enteral, e.g. oral, or parenteral administration. Accordingly, tablets or gelatin capsules are used that contain the active ingredient together with diluents, typically lactose, dextrose, saccharose, mannitol, sorbitol, cellulose and/or lubricants, e.g. diatomaceous earth, talcum, stearic acid or salts thereof, such as magnesium stearate or calcium stearate, and/or polyethylene glycol. Tablets may also contain binders, typically magnesium aluminium silicate, starches, typically corn starch, wheat starch, rice starch or arrow root starch, gelatin, tragacanth, methylcellulose, sodium carboxymethylcellulose and/or polyvinylpyrrolidone, and, if desired, disintegrators, typically starches, agar, alginic acid or a salt thereof, e.g. sodium alginate, and/or effervescent mixtures, or absorbents, colourants, flavourings and sweeteners.

Thus in another aspect, the invention relates to a pharmaceutical composition comprising a compound of the present invention (and optionally other therapeutically effective agents), and a pharmaceutically acceptable carrier for the treatment or prevention of artheriosclerosis or for the reduction of cholesterol levels.

The terms "effective amount" and "therapeutically effective amount" mean that amount of a compound of the present invention (and optionally other therapeutically effective agents), that will elicit a biological or medical response of a tissue, system, animal or mammal, which includes alleviation of the symptoms of the condition or disease being treated and the prevention, slowing or halting of progression of one or more conditions, for example atherosclerosis, hypercholesterolemia.

The pharmaceutical compositions so obtained which, if desired, contain further pharmacologically active substances, are prepared in a manner known per se by conventional mixing, granulating, sugar-coating, solution or lyophilising methods and contain from about 0.1% to 100%, preferably from about 1% to about 50%, lyophilisate to about 100%, of active ingredient.

The compounds, compositions and treatments of the present invention can be administered by any suitable means which produce contact of these compounds with the site of action in the body, for example in the plasma, liver or small intestine of a mammal or human. Thus the novel compounds of the present invention may also be used in the form of compositions for parenteral, oral, transdermal administration or infusion solutions. Such solutions are preferably isotonic aqueous solutions or suspension which, e.g. in the case of lyophilised compositions that contain the active ingredient by itself or together with a carrier, such as mannitol, can be prepared before use. The pharmaceutical compositions can be sterilised and/or can contain excipients, typically preservatives, stabilisers, wetting agents and/or emulsifiers, solubilisers, salts for regulating the osmotic pressure and/or buffers.

In yet a further aspect, the invention relates to a kit comprising an effective amount of a compound of the present invention in a pharmaceutically acceptable carrier (and optionally an effective amount of another therapeutically effective agent), optionally in separate compartments.

The following non-limiting Examples illustrate the above-described invention in more detail.

### EXAMPLES

***Materials and Methods:*** Reactions in anhydrous solvents were all performed using oven dried glassware under an atmosphere of argon. Reagent grade solvents were all purchased from chemical companies and used without prior purification. Tert-Butyl hypochlorite was prepared according to literature procedures (Mintz, M. J.; Walling, C. Organic Syntheses,; Wiley: New York, 1983; Collect. Vol. V, p 183). For chromatic purification, technical grade solvents were distillated prior to use. TLC was performed using Machery-Nagel Alugram Sil G/UV₂₅₄ TLC plates and visualized with ultraviolet light at 254 nm and 12 g phosphor molybdic acid in 250 mL EtOH or 10% H₂SO₄ in MeOH (v/v). Chromatographic purification of products was accomplished by flash chromatography on silica gel 60 (230 - 400 mesh, 0.04 - 0.063 mm) from Merck at rt and 0.3 - 0.5 mbar air pressure; fractions containing product were pooled, the solvents were evaporated under reduced pressure and the residue was dried under high vacuum to give the product. NMR spectra were recorded on a Varian Mercury 300MHz apparatus operating at 300 MHz and 75 MHz for ¹H and ¹³C, respectively, and chemical shifts (δ) were referenced to the internal solvent signals. Melting points were measured on a Büchi 510 apparatus in open capillaries and all melting points are uncorrected. IR-Spectra were recorded in CHCl₃ on a Perkin Elmer Spectrum RX I FT-IR apparatus (thin films on NaCl plates) and are reported as absorption maxima in cm⁻¹ (data not submitted). High resolution matrix-assisted laser desorption ionization mass spectrometry (MALDI-MS) and electrospray ionization (ESI-MS) were performed by the mass spectrometry service of the LOC at the ETH, Zürich and were recorded in positive ion mode.

### Example 1: Synthesis of (4R,5S)-4-(4-(benzyloxy)phenyl)-oxazolidin-2-one-5-carboxylic acid 4.

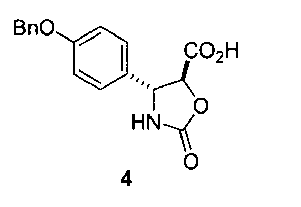

### (a) Synthesis of (E)-Isopropyl 3-(4-(benzyloxy)phenyl)acrylate 2

To a suspension of 4-hydroxy-cinnamic acid 1 (26.520 g, 162.0 mmol) in iPrOH (200 mL) at 0 °C was added dropwise thionyl chloride (12.0 mL, 162.0 mmol). The icebath was removed and the solution was heated at reflux for 5 h under Argon. After cooling to room temperature, Na₂CO₃ (17.17 g, 162 mmol) was added to the reaction mixture, followed by H₂O (200 mL). The mixture was extracted with EtOAc (3 x 100 mL), washed with saturated solution of NaHCO₃ (2x50 mL) and brine (2 x 50 mL). The organic solution was dried over Na₂SO₄ and concentrated under vacuum, yielding a yellow oil. This residue was dissolved in dry DMF (200 mL), treated with K₂CO₃ (28.00 g, 202 mmol) and tetra-*n*-butylammonium iodide (3.00 g, 8.1 mmol). Benzyl bromide (19.3 mL, 162 mmol) was dropwise added over 5 min and the reaction mixture was stirred for 16 h at 23 °C. The resulting suspension was diluted with water (800 mL) and Et₂O (400 mL). The phases were separated and the aqueous phase was extracted with Et₂O (3x150 mL). The combined organic phases were washed with water (2x100 mL), brine (1x100 mL), dried over Na₂SO₄ and concentrated under vacuum. The residue was triturated with 5:1 hexane/Et₂O (100 mL) and the resulting colorless solid was filtered out and washed with 5:1 hexane/Et₂O (2x50 mL). The mother liquor was concentrated under vacuum and purified by flash chromatography (95:5 hexane/EtOAc) yielding a second crop of colorless (E)-Isopropyl 3-(4-(benzyloxy)phenyl)acrylate **2** as a solid (total, 37.890 g, 127.9 mmol, 79% yield two steps). Mp 61-62 °C (5:1 hexane-Et₂O); ¹H-NMR (CDCl₃, 300 MHz) δ 7.62 (d, *J* 16.2, 1H), 7.33-7.48 (m, 7H), 6.97 (d, *J* 8.70, 2H), 6.28 (d, *J* 16.2, 1H), 5.10 (m, 3H), 1.30 (d, *J* 6.3, 6H); ¹³C NMR (CDCl₃, 75 MHz) δ 166.70, 160.29, 143.81, 136.39, 129.59, 128.59, 128.08, 127.40, 116.38, 115.12, 70.09, 67.62, 22.12; HRMS (EI) found 296.1408, C₁₉H₂₀O₃⁺ requires 296.1412.

### (b) Synthesis of (2S, 3R)-Isopropyl 3-(4-(benzyloxy)phenyl)-3-(benzyloxycarbonylamino)-2-hydroxypropanoate 3a.

A 1L round-bottomed flask, equipped with a magnetic stir bar, was charged with NaOH (5.300 g, 130.5 mmol) and H₂O (400 mL). A 15 mL portion of this aqueous solution was used to dissolve the K₂OsO₂(OH)₄ (675 mg, 1.80 mmol) salt providing the pink catalyst solution. To this stirred solution, benzyl carbamate (21.150 g, 140 mmol) and n-PrOH (225 mL) were added. The mixture was sonicated until complete dissolution of the carbamate and was immersed in a room temperature water bath. Freshly prepared tert-butyl hypochlorite (16 mL, 137 mmol) was dropwise added with stirring over 15 min (Mintz, M. J. and Walling, C. Organic Syntheses,; Wiley: New York, 1983; Collect. Vol. V, p 183; Guigen, Li et al, Angew. Chem. Int. Ed. Engl. 1996, 35 (23/24), 2813-2817; Reddy, K., L. and Sharpless, K., B. J. Am. Chem. Soc. 1998, 120, 1207-1217). A solution of the cinnamate (13.320 g, 45.00 mmol) and (DHQD)₂PHAL (1.800 g, 2.25 mmol) in n-PrOH (225 mL) was added over 5 min using a dropping funnel, followed by the above described K₂OsO₂(OH)₄ solution. The reaction mixture turned green immediately after the addition of the catalyst and was stirred for 30 min at 20 °C, while the green solution became a light yellow heterogeneous mixture with the appearance of abundant precipitate. The reaction mixture was cooled to 0 °C for 1.5 h and the resulting off-white solid was collected by filtration, washed with 1:1 *n*-PrOH/H₂O (2 x 100 mL) and dried *in vacuo* to afford the pure expected product (2*S*,3*R*)-Isopropyl 3-(4-(benzyloxy)phenyl)-3-(benzyloxycarbonylamino)-2-hydroxypropanoate **3a** (16.450 g, 79%). The enantiomeric excess (>95%) was determined by ¹H NMR analysis of the corresponding Mosher's ester (data not submitted). Mp 141-142 °C (*n*-PrOH); [α]_{D}²⁰ -21.4 (c 0.52, CHCl₃); ¹H-NMR (CDCl₃, 300 MHz) δ 7.30-7.44 (m, 12H), 6.95 (d, *J* 6.90, 2H), 5.60 (br. d, *J* 9.30, 1H, NH), 5.22 (br. d, *J* 9.30, 1H, H₃), 5.10 (m, 5H), 4.40 (br. s, 1H, H₂), 3.16 (d, *J* 3.90, 1H, OH), 1.28 (d, *J* 6.30, 3H), 1.21 (d, *J* 6.30, 3H); ¹³C NMR (CDCl₃, 75 MHz) δ 172.21, 158.29, 155.47, 136.85, 136.23, 131.39, 128.51, 128.39, 128.01, 127.92, 127.36, 114.80, 73.47, 70.68, 69.91, 66.84, 55.76, 21.58, 21.36; HRMS (MALDI) found 486.1879, C₂₇H₂₉NO₆Na⁺ requires 486.1887.

### (c) Synthesis of (2R,3S)-Isopropyl 3-(4-(benzyloxy)phenyl)-3-(benzyloxycarbonylamino)-2-hydroxypropanoate 3b.

The title compound **3b** was prepared using the same procedure described for compound **3a,** but using (DHQ)₂PHAL as ligand. The reaction was run in 1 mmol scale and the starting material was consumed after 3 h stirring at 20 °C. The expected compound was obtained as a colorless solid (329 mg, 70%) with identical ¹H NMR to compound **3a.** The enantiomeric excess (>95%) was determined by ¹H NMR analysis of the corresponding Mosher's ester (data not submitted). [α]_{D}²⁰ +19.0 (c 0.52, CHCl₃)

### (d) Synthesis of (4R,5S)-4-(4-(benzyloxy)phenyl)-oxazolidin-2-one-5-carboxylic acid 4.

To a suspension of compound **3a** (or **3b** respectively) (16.330 g, 35.23 mmol) in dry MeOH (300 mL) was added solid K₂CO₃ (5.40 g, 38.76 mmol). The reaction mixture was heated at reflux for 2.5 h under argon. The resulting solution was cooled down to 20 °C and H₂O (3.00 mL, 166 mmol) was added. The mixture was heated at reflux for another 60 min and, after cooled to 20 °C, the solvent was removed *in vacuo.* The resulting off-white solid was dissolved in H₂O (350 mL) and washed with EtOAc (6x200 mL) to remove the benzyl alcohol. The aqueous phase was cooled to 0 °C and acidified with 3M HCl until pH 3. The resulting suspension was extracted with EtOAc (2x400 mL, 2x200 mL). Organics were washed with H₂O (1x50 mL), brine (1x50 mL), dried over Na₂SO₄, and concentrated in *vacuo.* The expected compound **4** was obtained as a colorless solid (10.660 g, 97%). Mp 162-164 °C; [α]_{D}²⁰ +62.9 (c 0.52, MeOH); ¹H-NMR (DMSO-d₆, 300 MHz) δ 13.40-13.80 (br. s, 1H, CO₂H), 8.39 (s, 1H, NH), 7.29-7.43 (m, 7H), 7.05 (d, 2H, *J* 8.70), 5.12 (s, 2H), 4.83 (d, *J* 5.10, 1H), 4.72 (d, *J* 5.10, 1H); ¹³C NMR (DMSO-*d₆*, 75 MHz) δ 170.07, 158.09, 157.13, 136.77, 132.45, 128.26, 127.66, 127.45, 127.32, 114.92, 79.00, 69.14, 57.91; HRMS (EI) found 313.0947, C₁₇H₁₅NO₅⁺ requires 313.0945.

### Example 2: Synthesis of (4R,5S)-4-(4-(Benzyloxy)phenyl)-5-(hydroxymethyl)oxazolidin-2-one 5.

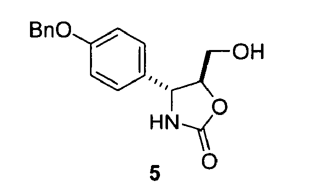

A solution of the acid **4** (10.140 g, 32.40 mmol) in MeOH (200 mL) was purged with argon and thionyl chloride (0.50 mL, 6.85 mL) was carefully added at 20 °C. The reaction mixture was stirred for 3 h at 20 °C. A stream of N₂ was bubbled through the solution for 30 min, and, then the solvent was removed under vacuum, whereupon the corresponding methylester (4*R*,5*S*)-Methyl 4-(4-(benzyloxy)phenyl)-oxazolidin-2-one-5-carboxylate was obtained as an off-white solid. Mp 128-129 °C (MeOH); ¹H-NMR (CDCl₃, 300

MHz) δ 7.25-7.41 (m, 9H), 7.00 (d, *J* 8.70), 5.79 (s, 1H, NH), 5.07 (s, 2H), 4.91 (d, *J* 5.10, 1H), 4.73 (d, *J* 5.10, 1H), 3.85 (s, 3H, OMe); HRMS (EI) found 327.1100, C₁₈H₁₇NO₅⁺ requires 327.1102.

To a suspension of the corresponding methyl ester (32.40 mmol) in ethanol (200 mL) was added at 20 °C NaBH₄ (1.85 g, 48.6 mmol) . The suspension was stirred for 90 min, at which point all solids were dissolved. To this solution was added NH₄Cl (7.00 g) and the EtOH was removed under vacuum. The residue was suspended in H₂O (200 mL) and the resulting off-white solid was collected by filtration. The solid was washed with H₂O (4x50 mL) and dried over P₂O₅ under vacuum for 24 h, to afford the pure target compound 5 (8.820 g, 91% two steps). Mp 161-162 °C; [α]_{D}²⁰ +25.6 (c 0.51, MeOH); ¹H-NMR (DMSO-*d₆*, 300 MHz) δ 8.04 (s, 1H, NH), 7.24-7.45 (m, 7H), 7.04 (d, *J* 8.40, 2H), 5.20 (t, *J* 5.70, 1H, OH), 5.10 (s, 2H), 4.60 (d, *J* 6.00, 1H, H₄), 4.15 (m, 1H, H₅), 3.55 (m, 2H); ¹³C NMR (DMSO-*d₆*, 75 MHz) δ 157.88, 157.82, 136.78, 132.95, 128.22, 127.59, 127.39, 127.29, 114.85, 84.02, 69.08, 60.91, 56.12; HRMS (EI) found 299.1143, C₁₇H₁₇NO₄⁺ requires 299.1153.

### Example 3: Synthesis of N-aryl oxazolidinones 6 - 10:

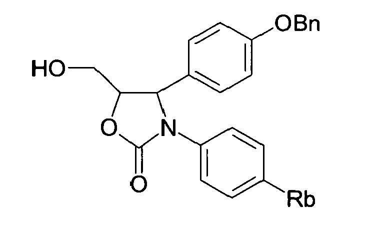

| **compound** | **Rb** |
|---|---|
| **6** | **-H** |
| **7** | **-F** |
| **8** | **-CF₃** |
| **9** | **-CN** |
| **10** | **-CONH-nPr** |

### (a) General procedure:

A Schlenk tube was charged with oxazolidinone 5 (150 mg, 0.50 mmol), K₂CO₃ (138 mg, 1.00 mmol), CuI (10 mg, 0.05 mmol, 10 mol%) and filled with argon. N,N'-Dimethylethylenediamine (11 µL, 0.10 mmol, 20 mol%), the corresponding arylbromide of choice (0.50 mmol) and dioxane (1.0 mL) were added under argon. The reaction mixture was stirred at 100 °C for 16 h. The resulting suspension was allowed to reach room temperature and filtered through a pad of Silica gel, eluting with EtOAc. The filtrate was concentrated *in vacuo* and the residue was purified by flash chromatography (1:1 hexane/EtOAc) to obtain the corresponding N-aryl oxazolidinones 6 - 10.

### (b) Synthesis of (4R,5S)-4-(4-(Benzyloxy)phenyl)-5-(hydroxymethyl)-3-phenyloxazolidin-2-one 6.

Colorless solid (162 mg, 0.43 mmol, 86% yield); Mp 150-152 °C; [α]_{D}²⁷ -13.4(c 0.50, CHCl₃) ; ¹H-NMR(CDCl₃, 300 MHz) δ 7.39-7.25 (m, 11H), 7.09-7.02 (m, 1H), 6.93 (d, *J* 8.7, 2H), 5.30 (d, *J* 6.9, 1H, H₄), 5.01 (s, 2H, PhCH₂O), 4.41-4.37 (m, 1H, H₅), 4.10-3.95 (m, 1H, CH₂OH), 3.82-3.75 (m, 1H, CH₂OH), 2.20 (br. s, 1H, OH); ¹³C NMR (CDCl₃, 75 MHz) δ 158.92, 155.66, 136.73, 136.47, 129.93, 128.77, 128.56, 128.05, 127.85, 127.43, 124.84, 121.49, 115.53, 82.07, 70.09, 61.46, 61.34; HRMS (EI) found 375.1467, C₂₃H₂₁NO₄⁺ requires 375.1466.

### (c) Synthesis of (4R,5S)-4-(4-(Benzyloxy)phenyl)-3-(4-fluorophenyl)-5-(hydroxymethyl)oxazolidin-2-one 7.

Colorless solid (157 mg, 0.40 mmol, 80% yield); [α]_{D}²⁷ -14.1° (c 0.50, CHCl₃); Ref -16° (c1.54, CHCl₃); ¹H-NMR (CDCl₃, 300 MHz) 7.42-7.19 (m, 9H), 7.00-6.90 (m, 4H), 5.23 (d, *J* 6.5, 1H, H₄), 5.02 (s, 2H, PhCH₂O), 4.41-4.36 (m, 1H, H₅), 3.99 (m, 1H, CH₂OH), 3.74 (m, 1H, CH₂OH), 2.20 (m, 1H, OH).

### (d) Synthesis of (4R,5S)-4-(4-(Benzyloxy)phenyl)-5-(hydroxymethyl)-3-(4-(trifluoromethyl)phenyl)oxazolidin-2-one 8.

Colorless solid (178 mg, 0.40 mmol, 80% yield); Mp 126-128 °C; [α]_{D}²⁷ +4.2(c 0.50, CHCl₃); ¹H-NMR (CDCl₃, 300 MHz) δ 7.52-7.22 (m, 12H), 6.95 (d, *J* 8.7, 2H), 5.34 (d, *J* 6.6, 1H, H₄), 5.02 (s, 2H, PhCH₂O), 4.41 (m, 1H, H₅), 4.05 (m, 1H, CH₂OH), 3.82 (m, 1H, CH₂OH), 2.11 (m, 1H, OH); ¹³C NMR (CDCl₃, 75 MHz) δ 159.13, 155.16, 139.87, 136.34, 129.19, 128.57, 128.10, 127.60, 127.43, 125.93, 120.43, 115.79, 82.12, 70.14, 61.29, 60.92; ¹⁹F-NMR (CDCl₃, 282 MHz) δ -62.1 (CF₃); HRMS (EI) found 443.1339, C₂₄H₂₀F₃NO₄⁺ requires 443.1339.

### (e) Synthesis of 4-((4R,5S)-4-(4-(Benzyloxy)phenyl)-5-(hydroxymethyl)-oxazolidin-2-one-3-yl)benzonitrile 9.

Colorless needles (152 mg, 0.38 mmol, 76% yield); Mp 140-142 °C; [α]_{D}²⁷ -13.4 (c 0.50, CHCl₃); ¹H-NMR(CDCl₃, 300 MHz) δ 7.51 (m, 4H), 7.39-7.30 (m, 5H), 7.21 (d, *J* 9.0, 2H), 6.96 (d, *J* 8.80, 2H), 5.34 (d, *J* 6. 0, 1H, H₄), 5.02 (s, 2H, PhCH₂O), 4.39 (m, 1H, H₅), 4.10-4.00 (m, 1H, CH₂OH), 3.82-3.75 (m, 1H, CH₂OH), 2.85 (br. s, 1H, OH); ¹³C NMR (CDCl₃, 75 MHz) δ 159.20, 154.88, 140.87, 136.30, 132.80, 128.94, 128.59, 128.12, 127.49, 127.42, 120.38, 118.51, 115.90, 107.35, 82.19, 70.16, 61.22, 60.76; HRMS (EI) found 400.1419, C₂₄H₂₀N₂O₄⁺ requires 400.1418.

### (f) Synthesis of 4-((4R,5S)-4-(4-(benzyloxy)phenyl)-5-(hydroxymethyl)-2oxooxazolidin-3-yl)-N-propylbenzamide 10.

Colorless solid (220 mg, 0.48 mmol, 96%). ¹H-NMR (CDCl₃, 300 MHz) δ 7.60 (d, *J* 9.0, 2H), 7.41-7.31 (m, 7H), 7.20 (d, *J* 8.7, 2H), 6.92 (d, *J* 8.7, 2H), 6.20 (broad t, *J* 6.0, 1H, NH), 5.35 (d, *J* 6.3, 1H, H₄), 5.00 (s, 2H, PhCH₂O), 4.38 (m, 1H, H₅), 4.02 (m, 1H, CH₂OH), 3.80 (m, 1H, CH₂OH), 3.36 (q, *J* 6.9, 2H, CH₂-N), 2.78 (m, 1H, OH), 1.61 (m, 2H), 0.95 (t, 3H, *J* 7.2, CH₃).

### Example 4: General procedure for the preparation of compounds 11-15 by catalytic hydrogenation

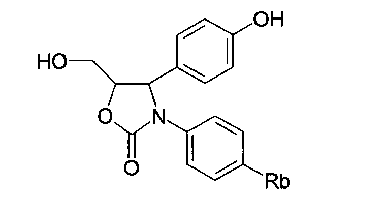

| **Compound** | **Rb** |
|---|---|
| **11** | **-H** |
| **12** | **-F** |
| **13** | **-CF₃** |
| **14** | **-CN** |
| **15** | **-CONH-nPr** |

### (a) General procedure:

The corresponding benzyl protected intermediate (6-10, 0.12-0.15 mmol) was suspended in EtOH (2.00 mL) and 10% Pd-C (10 mg) was added. The atmosphere was changed to hydrogen (1 atm) and the suspension was vigorously stirred for 6 h at 23 °C. The suspension was filtered through a short pad of silica gel and celite on top, eluting with EtOAc. The filtrate was concentrated, suspended in CH₂Cl₂/hexane and concentrated *in vacuo* to afford the expected compound in almost quantitative yield.

### (b) Synthesis of (4R,5S)-5-(hydroxymethyl)-4-(4-hydroxyphenyl)-3-phenyloxazolidin-2-one 11.

¹H-NMR (acetone-*d₆*, 300 MHz) δ 7.55 (d, *J* 8.7, 2H), 7.30 (m, 4H), 7.05 (m, 1H), 6.84 (d, *J* 8.7, 2H), 5.40 (d, *J* 6.2, 1H, H₄), 4.37 (m, 1H, H₅), 4.00-3.90 (m, 2H, CH₂OH), 2.05 (m, 1H, OH); ¹³C-NMR (acetone-*d₆*, 75 MHz) δ 158.1, 155.6, 138.5, 130.4, 129.1, 128.6, 124.6, 121.6, 116.5, 82.9, 62.2, 61.6; HRMS (EI) found 285.0995, C₁₆H₁₅NO₄⁺ requires 285.0996.

### (c) Synthesis of (4R,5S)-3-(4-fluorophenyl)-5-(hydroxymethyl)-4-(4-hydroxyphenyl)oxazolidin-2-one 12.

¹H-NMR (DMSO-d₆, 300 MHz) δ 7.40 (m, 2H), 7.15 (m, 4H), 6.75 (d, *J* 8.3, 2H), 5.45 (d, *J* 6.1, 1H, H₄), 4.30 (m, 1H, H₅), 3.80-3.60 (m, 2H, CH₂OH); ¹³C-NMR (CD₃OD, 75 MHz) δ 160.0 (d, *J* 244), 158.6, 157.5, 134.1, 129.3, 129.0, 125.0 (d, *J* 8.0), 116.5, 115.9 (d, *J* 22.5), 83.8, 63. 1, 61.8; HRMS (EI) found 303.0904, C₁₆H₁₄FNO₄⁺ requires 303.0902.

### (d) Synthesis of (4R,5S)-5-(hydroxymethyl)-4-(4-hydroxyphenyl)-3-(4-(trifluoromethyl)phenyl)oxazolidin-2-one.

¹H-NMR (acetone-*d₆*, 300 MHz) δ 7.75 (d, *J* 8.7, 2H), 7.60 (d, *J* 8.7, 2H), 7.30 (d, *J* 8.3, 2H), 6.84 (d, *J* 8.3, 2H), 5.45 (d, *J* 6.1, 1H, H₄), 4.40 (m, 1H, H₅), 4.00-3.80 (m, 2H, CH₂OH), 2.05 (m, 1H, OH); ¹³C-NMR (acetone-*d₆*, 75 MHz) δ 158.3, 155.3, 142.0, 129.8, 128.5, 126.3, 126.2, 120.8, 116.7, 83.09, 62.1, 61.3; HRMS (EI) found 353.0872, C₁₇H₁₄F₃NO₄⁺ requires 353.0870.

### (e) Synthesis of 4-((4R,5S)-5-(hydroxymethyl)-4-(4-hydroxyphenyl)-2-oxooxazolidin-3-yl)benzonitrile 14.

¹H-NMR (acetone-*d₆*, 300 MHz) δ 8.56 (s, 1H), 7.80-7.60 (m, 4H), 7.28 (d, *J* 8.7, 2H), 6.85 (d, *J* 8.7, 2H), 5.47 (d, *J* 5.5, 1H, H₄), 4.51 (m, 1H, H₅), 4.40 (m, 1H), 4.00-3.80 (m, 2H, CH₂OH); ¹³C-NMR (acetone-*d₆*, 75 MHz) δ 158.6, 155.4, 142.8, 133.5, 130.0, 128.7, 121.1, 121.2, 116.9, 107.3, 83.3, 62.3, 61.4; HRMS (EI) found 310.0950, C₁₇H₁₄N₂O₄⁺ requires 310.0949.

### (f) Synthesis of 4-((4R,5S)-5-(hydroxymethyl)-4-(4-hydroxyphenyl)-2-oxooxazolidin-3-yl)-N-propylbenzamide 15.

¹H-NMR (acetone-*d₆*, 300 MHz) δ 7.77 (d, *J* 8.7, 2H), 7.72 (broad s, 1H), 7.57 (d, *J* 8.7, 2H), 7.26 (d, *J* 8.4, 2H), 6.83 (d, *J* 8.4, 2H), 5.46 (d, *J* 5.5, 1H, H₄), 4.38 (m, 1H, H₅), 3.90 (m, 2H, CH₂OH), 3.30 (q, *J* 6.9, 2H, CH₂-N), 1.57 (m, 2H), 0.89 (t, 3H, *J* 7.2, CH₃); ¹³C-NMR (acetone-*d₆*, 75 MHz) δ 166.9, 158.6, 155.7, 141.2, 131.1, 130.3, 128.8, 128.4, 120.7, 116.8, 83.2, 62.3, 61.6, 42.2, 23.5, 11.7; HRMS (EI) found 370.1523, C₂₀H₂₂N₂O₅⁺ requires 370.1524.

### Example 5: (4R,55)-4-(4-(benzyloxy)phenyl)-5-((4-fluorophenoxy)methyl)-3-(4-fluorophenyl)oxazolidin-2-one 16.

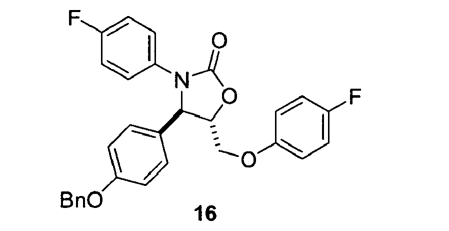

A Schlenk tube was charged with alcohol **7** (196 mg, 0.50 mmol), K₂CO₃ (138 mg, 1.00 mmol), CuI (10 mg, 0.05 mmol, 10 mol%) and filled with argon. *N,N'*-Dimethylethylenediamine (11 µL, 0.10 mmol, 20 mol%), then 1-bromo-4-fluorobenzene (77 µL, 0.70 mmol) and dioxane (1.0 mL) were added under argon. The reaction mixture was stirred at 100 °C for 16 h. The resulting suspension was allowed to reach room temperature and filtered through a pad of Silica gel, eluting with EtOAc. The filtrate was concentrated *in vacuo* and the residue was purified by flash chromatography (7:3 hexane/EtOAc). ¹H-NMR (CDCl₃, 300 MHz) δ 7.50-7.30 (m, 7H), 7.22 (d, *J* 8.7, 2H), 7.02-6.80 (m, 8H), 5.32 (d, *J* 6.6, 1H, H₄), 5.02 (s, 2H, PhCH₂O), 4.61 (m, 1H, H₅), 4.20 (m, 2H, CH₂O) ; ¹³C-NMR (CDCl₃, 75 MHz) δ 161.2, 159.2, 159.1, 158.0, 156.0, 155.0, 154.0, 136.3, 132.8, 129.6, 128.6, 128.1, 127.7, 127.4, 123.2, 123.1, 116.2, 115.8, 115.8, 115.7, 115.5, 79.5, 70.1, 67.7, 62.6; ¹⁹F-NMR (CDCl₃, 282 MHz) δ -117.3, -122.6; HRMS (MALDI) found 487.1598, C₂₉H₂₃F₂NO₄⁺ requires 487.1590.

### Example 6: (4R,5S)-5-((4-Fluorophenoxy)methyl)-3-(4-fluorophenyl)-4-(4-hydroxyphenyl)oxazolidin-2-one 17.

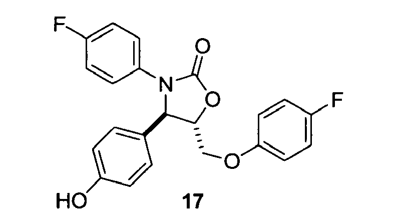

To a suspension of compound **16** (59 mg, 0.12 mmol) in EtOH (2.0 mL) was added 10% Pd-C (10 mg). The atmosphere was changed to hydrogen (1 atm) and the suspension was vigorously stirred for 5 h at 23 °C. The suspension was filtered through a pad of celite eluting with EtOAc. The filtrate was concentrated and the residue purified by flash chromatography (7:3 hexane/EtOAc), affording the expected compound as a colorless solid (45 mg, 0.11 mmol, 94%). [α]_{D}²⁷ +57.0(c 0.60, CHCl₃); ¹H-NMR (CDCl₃, 300 MHz) δ 7.40-7.18 (m, 6H), 7.02-6.80 (m, 6H), 5.28 (d, *J* 6.6, 1H, H₄), 4.95 (s, 1H, OH), 4.61 (m, 1H, H₅), 4.20 (m, 2H, CH₂O); ¹³C-NMR (CDCl₃, 75 MHz) δ 160.5 (d, *J* 245), 156.4, 155.4, 154.0, 132.6, 129.1, 127.9, 123.5 (d, *J* 8), 116.3, 116.1 (d, *J* 23), 115.79, 115.75 (d, *J* 23), 115.68, 79.7, 67.7, 62.7; ¹⁹F-NMR (CDCl₃, 282 MHz) δ -116.45, -121.80; HRMS (EI) found 397.1121, C₂₂H₁₇F₂NO₄⁺ requires 397.1121.

### Example 7: ((4R,5S)-4-(4-(Benzyloxy)phenyl)-3-(4-fluorophenyl)-2-oxooxazolidin-5-yl)methyl 4-fluorophenylcarbamate 18.

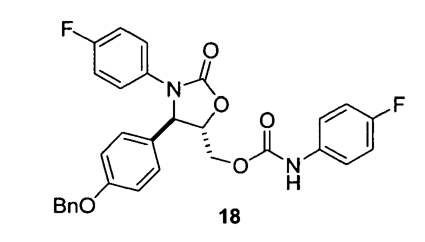

To a solution of the alcohol **7** (175 mg, 0.44 mmol) in CHCl₃ (3 mL) was added 4-fluorophenyl isocyanate (50 µL, 0.44 mmol), followed by pyridine (53 µL, 0.66 mmol) and the reaction mixture was heated at 60 °C for 12 h. After reached room temperature, the mixture was diluted with EtOAc (10 mL), washed with saturated solution of CuSO₄ (2x3 mL), NH₄Cl solution (1x3 mL) and brine (1x3 mL). The organic phase was dried over Na₂SO₄, concentrated *in vacuo* and purified by flash chromatography (65:35 hexane/EtOAc), affording the expected compound as a colorless crystalline solid (152 mg, 0.29 mmol, 65%). ¹H-NMR (CDCl₃, 300 MHz) δ 7.40-7.20 (m, 10H), 6.95 (m, 7H), 5.16 (d, *J* 6.6, 1H, H₄), 5.01 (s, 2H, PhCH₂O), 4.60-4.40 (m, 3H); ¹⁹F-NMR (CDCl₃, 282 MHz) δ - 116.29, -118.20; HRMS (MALDI) found 553.1537, C₃₀H₂₄F₂N₂O₅Na⁺ requires 553.1546.

### Example 8: ((4R,5S)-3-(4-Fluorophenyl)-4-(4-hydroxyphenyl)-2-oxooxazolidin-5-yl)methyl 4-fluorophenylcarbamate 19.

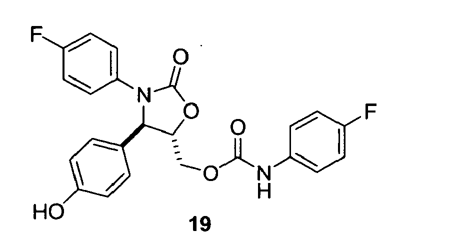

Using the same procedure as for compound **17,** but from **18** (120 mg, 0.23 mmol), compound **19** (95 mg, 0.22 mmol, 94%) was obtained as a colorless solid. [α]_{D}²⁷ +47.4(c 0.26, acetone); ¹H-NMR (DMSO-*d₆,* 300 MHz) δ 9.90 (s, 1H), 9.60 (broad s, 1H), 7.50-7.36 (m, 4H), 7.22 (d, *J* 8.7, 2H), 7.15 (m, 5H), 6.71 (d, *J* 8.4, 2H), 5.32 (d, *J* 6.6, 1H, H₄), 4.61 (m, 1H, H₅), 4.42 (m, 2H, CH₂O); ¹³C-NMR (DMSO-*d₆*, 75 MHz) δ 158.7 (d, *J* 241), 157.6 (d, *J* 239), 157.5, 154.3, 152.9, 134.9, 133.0, 128.3, 127.2, 123.8, 119.9, 115.6, 115.22 (d, *J* 22.5), 115.16 (d, *J* 22.5), 79.2, 63.3, 60.8;¹⁹F-NMR (DMSO-*d*₆, 282 MHz) δ -117.21, -120.15; HRMS (MALDI) found 463.1076, C₂₃H₁₈F₂N₂O₅Na⁺ requires 463.1076.

### Example 9: (4R,5R)-5-(Azidomethyl)-4-(4-(benzyloxy)phenyl)-3-(4-fluorophenyl)oxazolidin-2-one 20.

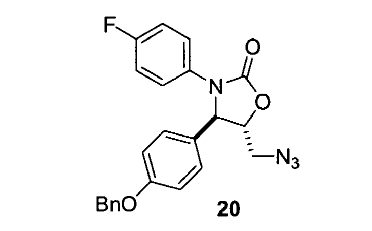

To a solution of the alcohol 7 (990 mg, 2.51 mmol) in THF (10 mL) was added PPh₃ (1.310 g, 5.00 mmol) followed by CBr₄ (1.660 g, 5.00 mmol). The reaction mixture was stirred at 23 °C for 1 h, when TLC in 1:1 hexane/EtOAc showed no starting material. The mixture was diluted with Et₂O (10 mL), the solid was filtered out and washed with Et₂O (3x10 mL). The solvents were removed under vacuum and the residue purified by flash chromatography (8:2 hexane/EtOAc) to afford a colorless solid.

The solid (1.109 g) was added to a DMSO solution of NaN₃ (10 mL, 0.50M) and the resulting solution was stirred overnight (14 h) at 23 °C. The mixture was diluted with H₂O (70 mL) and extracted with 2:1 Et₂O/CH₂Cl₂ (3x50 mL). The combined organic layers were washed with brine (2x20 mL), dried over Na₂SO₄ and concentrated *in vacuo.* The residue was treated with Et₂O, obtaining colorless needles. The solid was collected by filtration, washed with Et₂O (2x5 mL) and dried under vacuum to afford compound 20 (947 mg, 2.27 mmol, 90% two steps). Mp 107-108 °C (Et₂O); [α]_{D}²⁵ +59.9 (c 0.50, CHCl₃); ¹H-NMR (CDCl₃, 300 MHz) δ 7.42-7.18 (m, 9H), 6.97 (m, 4H), 5.07 (d, *J* 6. 0, 1H, H₄), 5.02 (s, 2H, PhCH₂O), 4.45 (m, 1H, H₅), 3.75 (dd, *J* 13.5 and 4.20, 1H, CH₂N₃), 3.58 (dd, *J* 13.5 and 4.20, 1H, CH₂N₃); ¹³C NMR (CDCl₃, 75 MHz) δ 159.7 (d, *J* 245), 159.2, 154.7, 136.3, 132.6, 128.9, 128.6, 128.1, 127.8, 127.4, 123.4 (d, *J* 8), 115.71 (d, *J* 23), 115.68, 79.7, 70.1, 63.0, 51.9; ¹⁹F-NMR (CDCl₃, 282 MHz) δ -116.3; HRMS (MALDI) found 441.1330, C₂₃H₁₉FN₄O₃Na⁺ requires 441.1333.

### Example 10: 1-(4-fluorophenyl)-3-(((4R, 5R)-3-(4-fluorophenyl)-4-(4-hydroxyphenyl)-2-oxooxazolidin-5-yl) methyl)urea 21.

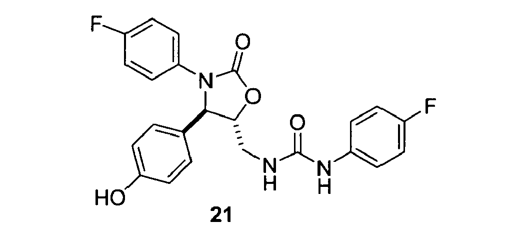

To a solution of azide **20** (80 mg, 0.19 mmol) in THF (1 mL) was added 1.00M THF solution of PMe₃ (300 µL, 0.30 mmol) under argon and the reaction mixture was stirred at 23 °C during 2 h. Then, H₂O (0.30 mL) was added and the mixture was stirred for another 2 h at 23 °C. The reaction mixture was diluted with water (3 mL) and extracted with CH₂Cl₂ (5x5 mL). Organics were washed with brine (1x3 mL) and dried over Na₂SO₄. After filtered the sodium sulfate off, the solvent was concentrated *in vacuo*, obtaining the amine intermediate as an oil that was used without purification in the next step (¹H-NMR (CDCl₃, 300 MHz) δ 7.42-7.20 (m, 9H), 6.90 (m, 4H), 5.16 (d, *J* 6.0, 1H, H₄), 5.00 (s, 2H), 4.36 (m, 1H), 3.20 (m, 2H), 3.17 (m, 2H), 1.40 (broad s, 2H)).

The crude amine intermediate was dissolved in CH₂Cl₂ (2 mL) and 1-fluoro-4-isocyanatobenzene (30 µL, 0.27 mmol) was added to the solution. After 10 min stirring at 23 °C, a TLC in 1:1 hexane/EtOAc, showed full conversion of the amine and the mixture was concentrated under vacuum. The residue was purified by flash chromatography (6:4 hexane/EtOAc) to obtain an off-white solid (¹H-NMR (CDCl₃, 300 MHz) δ 7.52 (s, 1H), 7.40-7.20 (m, 10H), 6.90 (m, 6H), 6.22 (broad, 1H), 5.38 (d, *J* 6.0, 1H, H₄), 5.01 (s, 2H), 4.46 (m, 1H), 4.10 (m, 1H), 3.45 (m, 1H)).

The so obtained off-white solid was disolved in EtOH/AcOH (1.00:0.20 mL) and 10% Pd-C (20 mg) was added. The atmosphere was changed to hydrogen (1 atm) and the suspension was vigorously stirred for 6 h at 23 °C. The suspension was filtered through a short pad of silica gel and celite on top, eluting with EtOAc. The filtrate was concentrated, suspended in CHCl₃ and concentrated *in vacuo.* The residue was treated with 1:1 CH₂Cl₂/hexane (3 mL), obtaining the compound **21** as a colorless solid which was filtered off (56 mg, 0.13 mmol, 71% three steps). [α]_{D}³⁰ +23.5 (c 0.26, acetone); ¹H-NMR (acetone-*d₆*, 300 MHz) δ 8.50 (s, 1H), 8.18 (s, 1H), 7.42 (m, 4H), 7.26 (m, 2H), 6.98 (m, 2H), 6.80 (m, 2H), 6.22 (broad s, 1H), 5.38 (d, *J* 6.0, 1H, H₄), 4.44 (m, 1H), 3.80-3.60 (m, 2H); ¹³C-NMR (acetone-*d₆*, 75 MHz) δ 159.9 (d, *J* 243), 158.6 (d, *J* 238), 158.3, 156.3, 155.6, 137.2, 134.6, 129.5, 128.9, 124.2 (d, *J* 8), 120.8 (d, *J* 8), 116.6, 115.9 (d, *J* 22.6), 115.7 (d, *J* 22.6), 81.8, 63.1, 41.8; HRMS (MALDI) found 462.1240, C₂₃H₁₉F₂N₃O₄Na⁺ requires 462.1236.

### Example 11: Synthesis of compounds 22-25

### (a) General procedure for the acylation of azide 20:

To a solution of azide **20** (80 mg, 0.19 mmol) in benzene (2 mL) was added 1.00M toluene solution of PMe₃ (250 µL, 0.25 mmol) under argon. The reaction mixture was stirred at 23 °C during 10 min, while observing a gas releasing (N₂). Then, a solution of the corresponding benzoic acid (0.21 mmol) in benzene (0.50 mL) was added and the reaction mixture was heated to 80 °C under argon for 12 h (Urpi, F. and Vilarrasa, J. Tetrahedron Lett. 1986, 27 (38), 4623-4624). After cooled down to room temperature, the reaction mixture was quenched with water (3 mL) and extracted with CH₂Cl₂ (4x5 mL). Organics were washed with sodium bicarbonate solution (1x3 mL), followed by brine (1x3 mL) and dried over Na₂SO₄. After filtered the sodium sulfate off, the solvent was concentrated *in vacuo* and the residue was purified by flash chromatography (6:4 to 1:1 Hex/EtOAc).

The crude intermediate was suspended in EtOH/AcOH (2.00:0.20 mL) and 10% Pd-C (15 mg) was added. The atmosphere was changed to hydrogen (1 atm) and the suspension was vigorously stirred for 6 h at 23 °C. The suspension was filtered through a short pad of silica gel and celite on top, eluting with EtOAc. The filtrate was concentrated, suspended in CH₂Cl₂/hexane and concentrated *in vacuo* to afford the expected compound.

### (b) Synthesis of N-(((4R, 5R)-3-(4-Fluorophenyl)-4-(4-hydroxyphenyl)-2-oxooxazolidin-5-yl)methyl)benzamide 22.

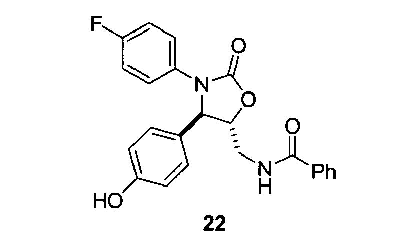

Colorless solid; 42 mg, 0.10 mmol, 86% (two steps). [α]_{D}³⁰ +33.1(c 0.28, acetone); ¹H-NMR (acetone-*d₆*, 300 MHz) δ 8.22 (broad s, 1H), 7.90 (m, 2H), 7.54-7.39 (m, 4H), 7.27 (d, *J* 8.40, 2H), 7.03 (m, 2H), 6.80 (d, *J* 8.40, 2H), 5.40 (d, *J* 5.9, 1H, H₄), 4.56 (m, 1H), 4.01-3.80 (m, 2H); ¹³C-NMR (acetone-*d₆*, 75 MHz) δ 168.0, 159.8 (d, *J* 243), 158.2, 155.4, 135.0, 134.5, 132.0, 129.5, 128.9, 128.8, 127.8, 124.0 (d, *J* 8), 116.5, 115.7 (d, *J* 23), 81.1, 63.5, 42.2; ¹⁹F-NMR (CDCl₃, 282 MHz) δ -117.95; HRMS (MALDI) found 429.1214, C₂₃H₁₉FN₂O₄Na⁺ requires 429.1021.

### (c) Synthesis of 4-fluoro-N-(((4R, 5R)-3-(4-fluorophenyl)-4-(4-hydroxyphenyl)-2-oxooxazolidin-5-yl)methyl)benzamide 23.

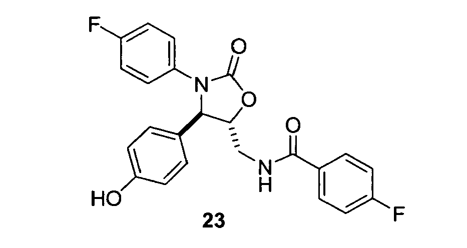

Off-white solid; 48 mg, 60% (two steps). [α]_{D}²⁷ +21.66 (c 2.00, acetone); ¹H-NMR (acetone-*d₆*, 300 MHz) δ 7.98 (m, 2H), 7.46 (m, 2H), 7.30 (m, 4H), 7.10 (m, 2H), 6.86 (m, 2H), 5.39 (d, *J* 6.0, 1H, H₄), 5.01 (s, 1H), 4.76 (m, 1H), 4.07-3.90 (m, 2H), 3.45 (m, 1H); ¹³C-NMR (acetone-*d₆*, 75 MHz) δ 168.0, 164.4 (d, *J* 249), 159.5 (d, *J* 244), 157.4, 155.7, 132.5, 129.7, 129.2 (d, *J* 9), 127.6, 123.7 (d, *J* 8), 115.2, 114.7 (d, *J* 23), 80.3, 63.4, 41.1; HRMS (MALDI) found 447.1123, C₂₃H₁₈F₂N₂O₄Na⁺ requires 447.1127.

### (d) Synthesis of N-(((4R, 5R)-3-(4-fluorophenyl)-4-(4-hydroxyphenyl)-2-oxooxazolidin-5-yl)methyl)picolinamide 24.

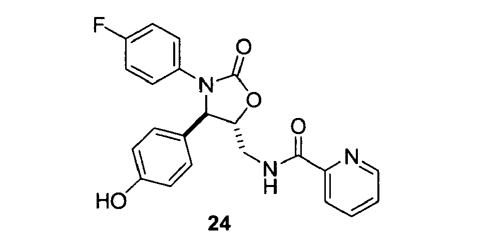

Colorless solid; 53 mg, 69% (two steps). ¹H-NMR (DMSO-*d₆*, 300 MHz) δ 9.52 (s, 1H), 9.18 (broad t, *J* 5.7, 1H, NH), 8.66 (d, *J* 4.5, 1H), 8.03 (m, 2H), 7.62 (m, 1H), 7.37 (m, 2H), 7.13 (m, 4H), 6.67 (d, *J* 8.7, 2H), 5.40 (d, *J* 5.4, 1H, H₄), 4.53 (m, 1H), 3.83-3.66 (m, 2H); ¹³C-NMR (DMSO-*d₆*, 75 MHz) δ 164.3, 158.6 (d, *J* 241), 157.2, 154.3, 149.2, 148.3, 137.7, 133.1, 127.9, 127.8, 126.6, 123.5 (d, *J* 8), 121.9, 115.6, 115.3 (d, *J* 22.5), 79.5, 62.1, 41.2; ¹⁹F-NMR (DMSO-*d₆*, 282 MHz) δ -117.33; HRMS (MALDI) found 408.1352, C₂₂H₁₉FN₃O₄⁺ requires 408.1354.

### (e) Synthesis of N-(((4R,5R)-3-(4-fluorophenyl)-4-(4-hydroxyphenyl)-2-oxooxazolidin-5-yl)methyl)-4-methoxybenzamide 25.

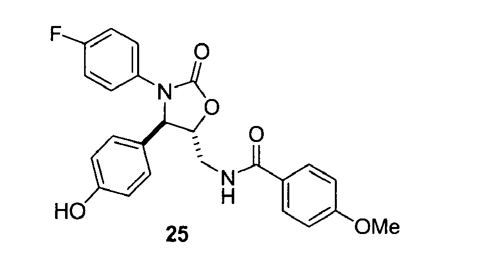

Colorless foam, 62 mg, 75% (two steps). [α]_{D}²⁷ +36.80 (c 2.50, acetone); ¹H-NMR (acetone-*d*₆, 300 MHz) δ 8.59 (s, 1H), 8.13 (broad t, *J* 5.7, 1H, NH), 7.92 (m, 2H), 7.43 (m, 2H), 7.27 (m, 2H), 7.00 (m, 4H), 6.82 (m, 2H), 5.40 (d, *J* 6.0, 1H, H₄), 4.57 (m, 1H), 3.97 (m, 1H), 3.86 (m, 4H) ; ¹³C-NMR (acetone-*d*₆, 75 MHz) δ 167.7, 162.9, 159.9 (d, *J* 241), 158.3, 155.4, 134.5, 129.8, 129.5, 128.8, 127.1, 124.0 (d, *J* 8), 116.5, 115.7 (d, *J* 22.5), 114.1, 81.3, 63.5, 55.6, 42.2; ¹⁹F-NMR (acetone-*d₆*, 282 MHz) δ - 117.92; HRMS (MALDI) found 459.1325, C₂₄H₂₁FN₂O₅Na⁺ requires 459.1327.

### Example 12: (4R, 5R)-3-(4-Fluorophenyl)-4-(4-hydroxyphenyl)-5-((4-phenyl-1H-1,2,3-triazol-1-yl)methyl)oxazolidin-2-one 26.

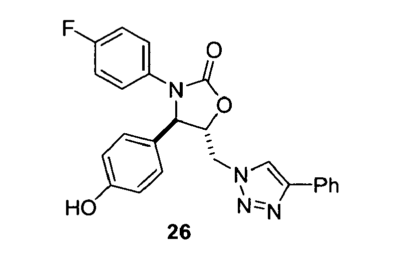

To a suspension of azide **20** (128 mg, 0.31 mmol) in H₂O/tBuOH (0.50:0.25 mL) was added phenylacetylene (34 µL, 0.31 mmol), followed by 1.00 M solution of sodium ascorbate (60 µL, 0.06 mmol) and 0.30 M solution of CuSO₄ (50 µL, 0.015 mmol). After 2 min of stirring at 23 °C the reaction mixture turned yellow and stirring was continued overnight (12 h) at the same temperature (Rostovtsev, V.-V. et al, Angew. Chem. Int. Ed. 2002, 41, 2596-2599; Tornøe, C.-W. et al, J. Org. Chem. 2002, 67, 3057-3064; Himo, F. et al, J. Am. Chem. Soc. 2005, 127, 210-216). The reaction mixture was extracted with EtOAc (3x5 mL). Organics were washed with brine and dried over Na₂SO₄. After filtered off the Na₂SO₄, the solvent was removed *in vacuo* and the residue purified by flash chromatography (6:4 hexane/EtOAc), affording a colorless solid (155 mg, 0.30 mmol, 96%: ¹H-NMR (CDCl₃, 300 MHz):δ 8.01 (s, 1H), 7.81 (m, 2H), 7.38 (m, 8H), 7.23 (s, 1H), 7.08 (m, 2H), 6.97-6.85 (m, 4H), 5.08 (d, *J* 6.6, 1H, H₄), 5.02 (s, 2H, PhCH₂O), 4.85 (m, 2H), 4.75 (m, 1H) .

The so obtained solid (74 mg, 0.14 mmol) was suspended in EtOH/AcOH (1.50:0.30 mL) and 10% Pd-C (30 mg) was added. The atmosphere was changed to hydrogen (1 atm) and the suspension was vigorously stirred for 12 h at 23 °C. The suspension was filtered through a short pad of silica gel and celite on top, eluting with EtOAc. The filtrate was concentrated, suspended in CHCl₃ and concentrated *in vacuo.* The expected compound **26** was obtained as a colorless solid (60 mg, 0.14 mmol, 100%). [α]_{D}²⁵ +54.2 (c 1.00, acetone); ¹H-NMR (acetone-*d₆*, 300 MHz) δ 8.47 (s, 1H), 7.89 (m, 2H), 7.47-7.27 (m, 7H), 7.03 (m, 2H), 6.84 (d, *J* 8.40, 2H), 5.45 (d, *J* 6.0, 1H, H₄), 5.06 (m, 2H), 4.91 (m, 1H), 2.80 (broad s, 1H); ¹³C-NMR (acetone-*d*₆, 75 MHz) δ 160.2 (d, *J* 240), 158.6, 154.76, 148.0, 134.1, 131.7, 129.4, 129.0, 128.8, 128.6, 126.1, 124.4 (d, *J* 8), 122.5, 116.7, 115.9 (d, *J* 23), 80.3, 63.4, 52.3; ¹⁹F-NMR (acetone-d₆, 282 MHz) δ -117.65; HRMS (MALDI) found 431.1513, C₂₄H₂₀FN₄O₃⁺ requires 431.1514.

### Example 13: (4R, 5R)-3-(4-fluorophenyl)-5-((4-(4-fluorophenyl)-1H-1,2,3-triazol-1-yl)methyl)-4-(4-hydroxyphenyl)oxazolidin-2-one 27.

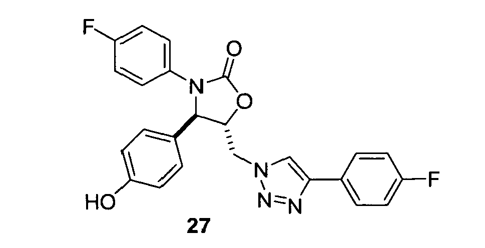

To a solution of azide **20** (80 mg, 0.19 mmol) in CH₃CN (0.3 mL) was added 1-ethynyl-4-fluorobenzene (25 µL, 0.21 mmol), followed by Et₃N (30 µL, 0.21 mmol). To this homogeneous solution was added H₂O (0.50 mL), CuI (4 mg, 0.02 mmol) and the resulting suspension was stirred at 23 °C overnight (14 h). The mixture was diluted with H₂O (5 mL) and extracted with EtOAc (3x6mL). Organics were washed with brine (1x3 mL), dried over Na₂SO₄ and filtered through a short pad of silica. The solvent was removed under vacuum, the residue was treated with EtOH (1 mL), and after removal the EtOH under vacuum, a colorless solid (99 mg, 97%) was obtained.

The so obtained solid (99 mg, 0.19 mmol) was suspended in EtOH/AcOH (2:0.20 mL) and 10% Pd-C (20 mg) was added. The atmosphere was changed to hydrogen (1 atm) and the suspension was vigorously stirred for 12 h at 23 °C. The suspension was filtered through a short pad of silica gel and celite on top, eluting with EtOAc. The filtrate was concentrated, suspended in CHCl₃ and concentrated *in vacuo.* The expected compound **27** was obtained as a colorless solid (76 mg, 0.17 mmol, 90% two steps). [α]_{D}²⁷ +65.8 (c 1.25, acetone); ¹H-NMR (acetone-*d₆*, 300 MHz) δ 8.62 (broad s, 1H), 8.47 (s, 1H), 7.92 (m, 2H), 7.39-7.18 (m, 6H), 7.00 (m, 2H), 6.84 (d, *J* 8.40, 2H), 5.44 (d, *J* 5.7, 1H, H₄), 5.07 (m, 2H), 4.92 (m, 1H); ¹³C-NMR (acetone- *d₆,* 75 MHz) δ 163.0 (d, *J* 244), 160.4 (d, *J* 244), 154.8, 147.1, 134.1, 129.0, 128.8, 128.2 (d, *J* 8), 124.4 (d, *J* 8), 124.4, 116.7, 116.3 (d, *J* 23), 115.9 (d, *J* 23), 80.2, 63.4, 52.3; HRMS (MALDI) found 449.1350, C₂₄H₁₉F₂N₄O₃⁺ requires 449.1347.

### Example 14: (4R,5R)-3-(4-fluorophenyl)-4-(4-hydroxyphenyl)-5-((4-(4-methoxyphenyl)-1H-1,2,3-triazol-1-yl)methyl)oxazolidin-2-one 28.

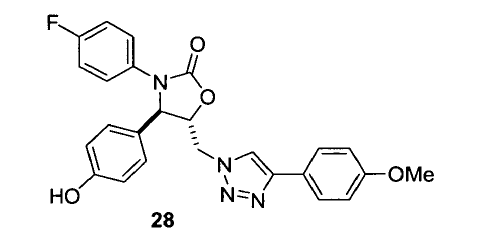

Following the same procedure than for compound **26,** but using 1-ethynyl-4-methoxybenzene (30 mg, 0.22 mmol) and azide **20** (80 mg, 0.19 mmol), compound **28** was obtained as a colorless solid (43 mg, 55% two steps). [α]_{D}²⁷ +74.6(c 1.50, acetone); ¹H-NMR (acetone-d₆, 300 MHz) δ 8.62 (s, 1H), 8.36 (s, 1H), 7.81 (d, *J* 9.0, 2H), 7.37 (m, 2H), 7.29 (d, *J* 8.7, 2H), 7.00 (m, 4H), 6.84 (d, *J* 8.40, 2H), 5.44 (d, *J* 5.5, 1H, H₄), 5.03 (m, 2H), 4.89 (m, 1H), 3.83 (s, 3H, OMe); ¹⁹F-NMR (acetone- *d₆,* 282 MHz) δ -117.49; ¹³C-NMR (acetone- *d₆,* 75 MHz) δ 160.3, 160.0 (d, *J* 242), 154.8, 148.0, 134.1, 129.0, 128.9, 127.5, 124.45 (d, *J* 8), 124.3, 121.6, 116.7, 115.9 (d, *J* 22.5), 114.9, 80.3, 63.4, 55.5, 52.2; HRMS (MALDI) found 461.1614, C₂₅H₂₂FN₄O₄⁺ requires 461.1620.

### Example 15: (4R, 5R)-3-(4-fluorophenyl)-4-(4-hydroxyphenyl)-5-((5-phenyl-1H-1,2,3-triazol-1-yl)methyl)oxazolidin-2-one 29

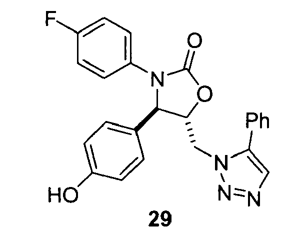

To a solution of azide **20** (80 mg, 0.19 mmol) in benzene (2 mL) was added (Ph₃P)₂RuCp*Cl (10 mg, 0.01 mmol) and phenylacetylene (23 µL, 0.21 mmol) under argon (Chin, M. S. and Heinekey, D. M. J. Am. Chem. Soc. 1990, 112, 5166-75, Zhang, L. et al, J. Am. Chem. Soc. 2005, 127, 15998-99). The reaction mixture was stirred at 23 °C for 12 h. The mixture was concentrated under vacuum and the residue was purified by flash chromatography (6:4 Hex/EtOAc), obtaining an off-white solid (93 mg, 0.18 mmol, 94%; ¹H-NMR (CDCl₃, 300 MHz) δ 7.70 (s, 1H), 7.47 (m, 4H), 7.40-7.32 (m, 6H), 7.21 (m, 2H), 7.08 (d, *J* 8.7, 2H), 5.42 (d, *J* 5.7, 1H, H₄), 5.02 (s, 2H, PhCH₂O), 4.74 (m, 2H), 4.65 (m, 1H).

The so obtained solid (90 mg) was disolved in EtOH/AcOH (2.00:0.20 mL) and 10% Pd-C (20 mg) was added. The atmosphere was changed to hydrogen (1 atm) and the suspension was vigorously stirred for 6 h at 23 °C. The suspension was filtered through a short pad of silica gel and celite on top, eluting with EtOAc. The filtrate was concentrated, suspended in CHCl₃ and concentrated *in vacuo.* The residue was treated with Et₂O (3 mL), obtaining a colorless solid that was filtered off (62 mg, 0.14 mmol, 76% two steps). [α]_{D}²⁷ +21.8 (c 1.00, acetone); ¹H-NMR (acetone-*d₆*, 300 MHz) δ 7.76 (s, 1H), 7.50 (m, 5H), 7.36 (m, 2H), 7.18 (d, *J* 8.7, 2H), 7.03 (m, 2H), 6.81 (d, *J* 8.4, 2H), 5.48 (d, *J* 6. 0, 1H, H₄), 4.97 (m, 2H), 4.81 (m, 1H); ¹⁹F-NMR (acetone-*d₆*, 282 MHz) δ -117.72; ¹³C NMR (acetone-*d₆*, 75 MHz) δ 163.0 (d, *J* 243), 158.5, 154.6, 139.5, 134.1, 133.4, 130.1, 129.7, 129.6, 128.9, 128.7, 127.5, 124.4 (d, *J* 8), 116.6, 115.7 (d, *J* 22.5), 80.1, 63.6, 50.3; HRMS (MALDI) found 431.1507, C₂₄H₂₀FN₄O₃⁺ requires 431.1514.

### Example 16: (4R,5R)-3-(4-fluorophenyl)-5-((5-(4-fluorophenyl)-1H-1,2,3-triazol-1-yl)methyl)-4-(4-hydroxyphenyl)oxazolidin-2-one 30

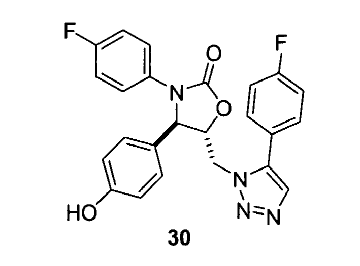

Following the same procedure than for compound **29,** but using 1-ethynyl-4-fluorobenzene (25 µL, 0.21 mmol) and azide **20** (80 mg, 0.19 mmol), compound **30** was obtained as a colorless solid after trituration with Et₂O (42 mg, 50% two steps). [α]_{D}²⁸ +30.6 (c 0.25, acetone); ¹H-NMR (DMSO-*d₆*, 300 MHz) δ 9.62 (broad s, 1H), 7.90 (s, 1H), 7.55 (m, 2H), 7.30 (m, 4H), 7. 12 (m, 4H), 6.70 (d, *J* 8.4, 2H), 5.37 (d, *J* 6.3, 1H, H₄), 4.98-4.83 (m, 2H), 4.74 (m, 1H); ¹³C NMR (DMSO-*d₆*, 75 MHz) δ 162.4 (d, *J* 246), 160.4, 157.5, 153.8, 137.3, 132.9, 131.0 (d, *J* 8), 129.1, 128.2, 126.8, 124.0 (d, *J* 8), 122.6, 115.9 (d, *J* 22.5), 115.6, 115.2 (d, *J* 22.5), 79.2, 62.2, 49.4; HRMS (MALDI) found 449.1345, C₂₄H₁₉F₂N₄O₃⁺ requires 449.1347.

### Example 17: (4R,5R)-3-(4-fluorophenyl)-4-(4-hydroxyphenyl)-5-((5-(4-methoxyphenyl)-1H-1,2,3-triazol-1-yl)methyl)oxazolidin-2-one 31

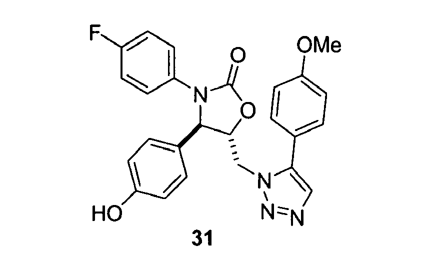

Following the same procedure than for compound **29,** but using 1-ethynyl-4-methoxybenzene (30 mg, 0.22 mmol) and azide **20** (80 mg, 0.19 mmol), compound **31** was obtained as a colorless solid after trituration with Et₂O (40 mg, 46% two steps). [α]_{D}²⁸ +28.6 (c 0.26, acetone); ¹H-NMR (acetone-*d₆*, 300 MHz) δ 7.71 (s, 1H), 7.45-7.34 (m, 4H), 7.19 (d, *J* 8.4, 2H), 7.03 (m, 4H), 6.83 (d, *J* 8.4, 2H), 5.48 (d, *J* 6.0, 1H, H₄), 4.95 (m, 2H), 4.80 (m, 1H), 3.85 (s, 3H); ¹³C NMR (acetone-d₆, 75 MHz) δ 161.3, 160.0 (d, *J* 241), 158.6, 154.8, 139.4, 134.2, 133.2, 131.1, 129.0, 128.7, 124.5 (d, *J* 8), 119.4, 116.6, 115.9 (d, *J* 22.5), 115.1, 80.2, 63.7, 55.7, 50.3; HRMS (MALDI) found 461.1543, C₂₅H₂₂FN₄O₄⁺ requires 461.1547.

### Example 18: ((4R,5S)-4-(4-(benzyloxy)phenyl)-5-(hydroxy-methyl)-2-oxooxazolidin-3-yl)-N-(2-(dimethylamino)ethyl)-benzamide 32.

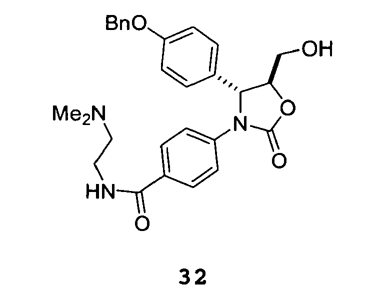

According to the general procedure given in Example 3(a), the oxazolidinone **5** was converted into compound **32,** which was a pale cream solid (231 mg, 0.47 mmol, 94% yield). ¹H-NMR (CDCl₃, 300 MHz) δ 7.65 (d, *J* 9.0, 2H), 7.43-7.31 (m, 7H), 7.20 (d, *J* 8.7, 2H), 6.92 (d, *J* 8.7, 2H), 6.80 (broad t, *J* 6.0, 1H, NH), 5.34 (d, *J* 6.6, 1H, H₄), 5.00 (s, 2H, PhCH₂O), 4.38 (m, 1H, H₅), 4.02 (m, 1H, CH₂OH), 3.80 (m, 1H, CH₂OH), 3.45 (m, 2H), 2.50 (m, 2H), 2.25 (s, 6H, CH₃); HRMS (MALDI) found 490.2331 C₂₈H₃₂N₃O₅⁺ requires 490.2337.

### Example 19: N-(2-(dimethylamino)ethyl)-4-((4R,5S)-5-(hydroxymethyl)-4-(4-hydroxyphenyl)-2-oxooxazolidin-3-yl)-benzamide 33

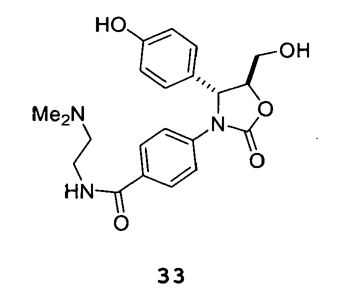

According to the general procedure given in Example 4(a), the benzyl-protected intermediate **32** was converted into the compound **33.** ¹H-NMR (acetone-*d₆*, 300 MHz) δ 7.75 (d, *J* 8.7, 2H), 7.61 (t, *J* 6.0, 1H), 7.55 (d, *J* 8.7, 2H), 7.25 (d, *J* 8.4, 2H), 6.81 (d, *J* 8.4, 2H), 5.45 (d, *J* 5.8, 1H, H₄), 4.38 (m, 1H, H₅), 3.95 (dd, *J* 12.0/3.0, 1H, CH₂OH), 3.84 (dd, *J* 12.0/3.0, 1H, CH₂OH), 3.45 (q, *J* 5.7, 2H, CH₂-N), 2.47 (t, *J* 6.6, 2H), 2.21 (s, 6H), 2.06 (m, 1H); ¹³C-NMR (acetone-*d₆*, 75 MHz) δ 166.6, 158.3, 155.4, 141.0, 130.7, 130.0, 128.6, 128.2, 120.5, 116.7, 83.1, 62.2, 61.5, 59.0, 45.5, 38.2; HRMS (EI) found 400.1868, C₂₁H₂₆N₃O₅⁺ requires 400.1815.

### Example 20: ((4R,5S)-4-(4-(benzyloxy)phenyl)-5-(hydroxy-methyl)-2-oxooxazolidin-3-yl)-N-(2-(trimethylamonium-iodide)ethyl)benzamide 34.

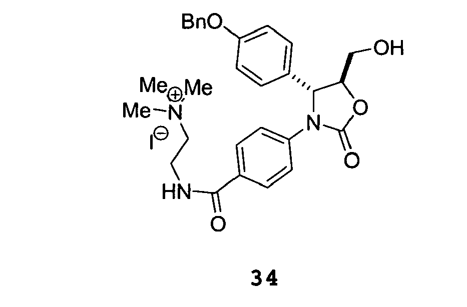

A solution of compound **32** (100mg, 0.20 mmol) and MeI (25 µL, 0.40 mmol) in i-PrOH (1 mL) was heated at 70 °C for 8 h. The reaction mixture was cooled down to room temperature and a yellow precipitate (116 mg, 0.18 mmol, 92%) was collected by filtration and triturated with Et₂O (3x1 mL) . ¹H-NMR (DMSO-*d*₆, 300 MHz) δ 8.66 (t, *J* 5.5, 1H), 7.74 (d, *J* 8.7, 2H), 7.55 (d, *J* 8.7, 2H), 7.42-7.31 (m, 7H), 6.98 (d, *J* 8.7, 2H), 5.48 (d, *J* 5.7, 1H, H₄), 5.36 (t, *J* 6.0, 1H), 5.03 (s, 2H), 4.36 (m, 1H, H₅), 3.75-3.63 (m, 4H), 3.45 (t, *J* 6.0, 2H), 3.10 (s, 9H); ¹³C-NMR (DMSO-*d₆*, 75 MHz) δ 165.7, 158.1, 154.3, 139.8, 136.7, 130.3, 128.7, 128.3, 127.9, 127.7, 127.6, 127.5, 119.8, 115.1, 81.9, 69.2, 63.7, 60.7, 59.8, 52.5, 33.7; HRMS (MALDI) found 504.2479, C₂₉H₃₄N₃O₅⁺ requires 504.2493.

### Example 21: ((4R,5S)-4-(4-hydroxyphenyl)-5-(hydroxymethyl)-2-oxooxazolidin-3-yl)-N-(2-(trimethylamoniumbromide)ethyl)-benzamide 35.

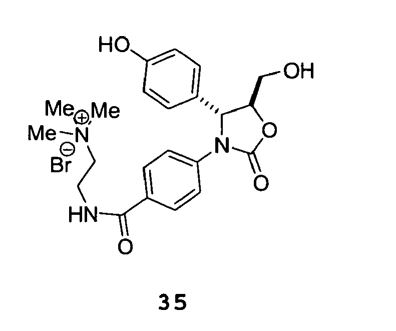

To a suspension of compound **34** (50 mg, 0.08 mmol) in CH₂Cl₂ (1 mL) was added 1M BBr₃ in CH₂Cl₂ (0.4 mL) at 5 °C and the reaction mixture was stirred overnight while the temperature increased to 23 °C. The reaction was quenched with MeOH (1 mL) and stirred for 30 min until a homogeneus solution was observed. The solvents were eliminated under vacuum, affording 35 as a colorless oil (25 mg, 0.06 mmol, 75%). ¹H-NMR (CD₃OD, 300 MHz) δ 7.75 (d, *J* 9.0, 2H), 7.53 (d, *J* 9.0, 2H), 7.17 (dd, *J* 6.6/2.0, 2H), 6.73 (dd, *J* 6.9/2.0, 2H), 5.38 (d, *J* 6.0, 1H, H₄), 4.39 (m, 1H, H₅), 3.92-3.74 (m, 4H), 3.56 (t, *J* 6.0, 2H), 3.20 (s, 9H); ¹³C-NMR (CD₃OD, 75 MHz) δ 169.1, 158.6, 141.5, 129.8, 129.3, 128.7, 128.6, 121.5, 116.6, 83.8, 65.6, 62.2, 61.9, 53.8, 34.9; HRMS (MALDI) found 414.2031, C₂₂H₂₈N₃O₅⁺ requires 414.2024.

### Example 22: 4-((4R,5S)-4-(4-(benzyloxy)phenyl)-5-(hydroxymethyl)-2-oxooxazolidin-3-yl)-N-(2-morpholinoethyl)-benzamide 36.

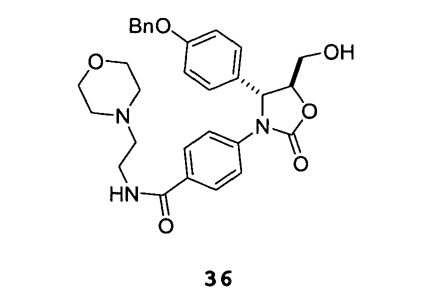

According to the general procedure given in Example 3(a), the oxazolidinone **5** was converted into compound **36,** which was a pale cream solid (273 mg, 0.47 mmol, 93% yield). ¹H-NMR (CDCl₃, 300 MHz) δ 7.63 (d, *J* 9.0, 2H), 7.46-7.32 (m, 7H), 7.21 (d, *J* 8.7, 2H), 6.93 (d, *J* 8.7, 2H), 6.72 (broad t, *J* 6.0, 1H, NH), 5.36 (d, *J* 6.6, 1H, H₄), 5.00 (s, 2H, PhCH₂O), 4.39 (m, 1H, H₅), 4.04 (dd, *J* 13.2/3.0, 1H, CH₂OH), 3.80 (dd, *J* 13.2/3.0, 1H, CH₂OH), 3.71 (t, *J* 4.5, 4H), 3.50 (m, 2H), 2.57 (t, *J* 6.0, 2H), 2.48 (t, *J* 4.5, 4H); ¹³C-NMR (CDCl₃, 75 MHz) δ 166.7, 159.0, 155.3, 139.6, 136.4, 129.9, 129.6, 128.6, 128.1, 127.7, 127.4, 120.3, 115.7, 82.3, 70.1, 66.9, 61.1, 60.8, 57.0, 53.4, 36.3; HRMS (MALDI) found 532.2360 C₃₀H₃₄N₃O₆⁺ requires 532.2369.

### Example 23: 4-((4R,5S)-5-(hydroxymethyl)-4-(4-hydroxy-phenyl)-2-oxooxazolidin-3-yl)-N-(2-morpholinoethyl)-benzamide 37.

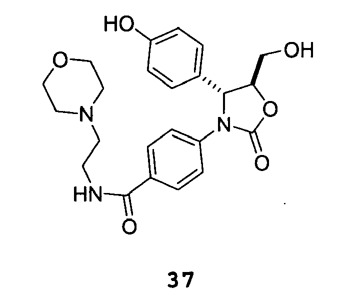

According to the general procedure given in Example 4(a), the benzyl-protected intermediate **36** was converted into the compound **37,** which was a colorless solid. ¹H-NMR (DMSO-d6, 300 MHz) δ 9.52 (s, 1H), 8.27 (t, *J* 5.1, 1H), 7.71 (d, *J* 9.0, 2H), 7.49 (d, *J* 9.0, 2H), 7.18 (d, *J* 8.4, 2H), 6.71 (d, *J* 8.4, 2H), 5.38 (d, *J* 5.7, 1H, H₄), 5.34 (t, *J* 5.4, 1H), 4.31 (m, 1H, H₅), 3.75-3.62 (m, 2H), 3.54 (t, *J* 4.8, 4H), 3.30 (m, 2H), 2.40 (m, 6H); ¹³C-NMR (DMSO-d6, 75 MHz) δ 165.2, 157.2, 154.4, 139.4, 129.6, 128.4, 127.8, 127.5, 119.7, 115.6, 82.0, 66.1, 60.8, 59.9, 57.3, 53.2, 36.5; HRMS (MALDI) found 442.2002 C₂₃H₂₈N₃O₆⁺ requires 442.1995.

### Example 24: 4-(2-(4-((4R,5S)-4-(4-(benzyloxy)phenyl)-5-(hydroxymethyl)-2-oxooxazolidin-3-yl)benzamido)ethyl)-4-methylmorpholin-4-ium iodide 38.

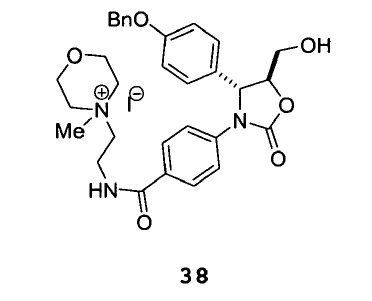

A solution of compound **36** (136 mg, 0.26 mmol) and MeI (50 µL, 0.80 mmol) in i-PrOH (2 mL) was heated at 70 °C for 8 h. The reaction mixture was cooled down to room temperature and a yellow precipitate (165 mg, 0.25 mmol, 95%) was collected by filtration and triturated with Et₂O (3x1 mL). ¹H-NMR (CD₃OD, 300 MHz) δ 7.75 (d, *J* 8.7, 2H), 7.55 (d, *J* 8.7, 2H), 7.40-7.28 (m, 7H), 6.97 (d, *J* 8.7, 2H), 5.44 (d, *J* 6.3, 1H, H₄), 5.03 (s, 2H), 4.42 (m, 1H, H₅), 4.00 (m, 4H), 3.90 (dd, *J* 12.9/3.3, 1H), 3.85-3.75 (m, 4H), 3.67-3.49 (m, 8H); ¹³C-NMR (DMSO-d6, 75 MHz) δ 166.0, 158.4, 154.6, 140.0, 136.9, 130.6, 128.9, 128.5, 128.1, 128.0, 127.9, 127.8, 120.0, 115.2, 82.0, 69.3, 62.0, 60.9, 59.8, 59.2, 46.6, 32.7, 25.5; HRMS (MALDI) found 546.2603, C₃₁H₃₆N₃O₆⁺ requires 546.2599.

### Example 25:

The compounds of the invention (commercially obtained or prepared according to Wu, G. Z. et al., J. Org. Chem. 1999, 64, 3714-3718) together with the glucuronide (the metabolite of ezetimibe, prepared according to Vaccaro and Davis, Bioorg. Med. Chem. Lett. 1998, 8, 313-318) as appropriate reference compounds were evaluated by well-established methods to determine their inhibition of cholesterol uptake in rabbit brush border membrane vesicles (BBMV) (Table 1). Briefly, the scavenger receptor-mediated uptake of radiolabelled cholesterol ester from the loaded donor particles into the BBMV bilayer was measured in the presence of various compounds of the invention and appropriate reference compounds (Kvaerno, L. et al, Angew. Chem. Int. Ed. 2004, 43, 4653-4656; Kvaerno, L. et al, Org. Lett. 2005, 7, 1145-1148).

| Compound | CAI (%) Inhibition |
|---|---|
| Ezetimibe | 15 ± 4 |
| 15 | 15 ± 4 |
| 29 | 15 ± 1 |
| 30 | 16 ± 1 |
| 32 | 30 ± 2 |
| 33 | 16 ± 2 |
| 34 | 29 ± 3 |
| 35 | 20 ± 1 |
| 36 | 33 ± 2 |
| 37 | 21 ± 3 |
| 38 | 24 ± 2 |

## Claims

1. A compound according to formula I or a pharmaceutically acceptable salt or solvate thereof, 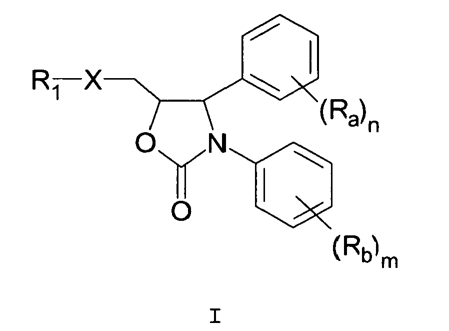 wherein
X represents a heteroatom linker selected from -O-, -NH-, -N=, -NH-CO-, -O-CO-NH-, -NH-CO-NH-, a heteroaryl group, or combinations thereof;
R₁ represents H, or R₂-substituted aryl Ar, wherein R₂ represents H, (C1-10)alkyl, -OR₃, -OCOR₃, -NR₃R₄, -NR₃(CO)R₄, - COOR₃, -CONR₃R₄, -CH=CHCOOR₃, -CF₃, -CN, -NO₂, or halogen, wherein R₃ and R₄ represent H or (C1-10)alkyl;
Rₐ represents independently of each other H, (C1-10)alkyl, -OR₅, -O(CO)R₅, -NR₅R₆, -NR₅(CO)R₆, halogen, -NO₂, or a polar moiety, wherein R₅ and R₆ represent H, (C1-10)alkyl, -Phe, or Bn;
R_{b} represents independently of each other H, (C1-10)alkyl, -OR₇, -O(CO)R₇, -NR₇R₈, -NR₇(CO)R₈, -COOR₇, -CONR₇R₈, -CH=CHCOOR₇, halogen, -CF₃, -CN, -NO₂, or a polar moiety, wherein R₇ and R₈ represent H or (C1-10)alkyl, and
n,m are independently of each other 0, 1 or 2.

2. A compound according to claim 1, wherein X is selected from - O-, -NH-, -NH-CO-, -O-CO-NH-, -NH-CO-NH-, or combinations thereof.

3. A compound according to claims 1 or 2, wherein when (Rₐ)ₙ equals -OBn in para position and (R_{b})ₘ equals -F in para position, -X-R₁ may not be -OH.

4. A compound according to claim 1, wherein X is a heteroaryl group.

5. A compound according to any preceding claim, wherein R₁ is H.

6. A compound according to any preceding claim, wherein R₁ is selected from R₂-substituted aryl Ar, wherein R₂ represents H, -OH, -O(C1-10)alkyl, -CF₃, -CN, or halogen.

7. A compound according to any preceding claim, wherein the heteroaryl group is a five- or six-membered ring having 1 to 3 heteroatoms selected from N, O, S and benzo-fused derivatives thereof.

8. A compound according to any preceding claim, wherein the heteroaryl group is selected from pyridine, pyrimidine, oxazole, thiazole, imidazole, triazole, pyrazole, and phthaleimide.

9. A compound according to any preceding claim, wherein R₂-substituted aryl Ar is a five- to ten-membered, carbocyclic or heterocyclic group, wherein R₂ is selected from H, (C1-10)alkyl, -OR₃, -NR₃R₄, -COOR₃, -CONR₃R₄, -CF₃, or halogen, wherein R₃ and R₄ represent H or (C1-10)alkyl.

10. A compound according to any preceding claim, wherein R₂-substituted aryl Ar is a five- or six-membered aromatic, carbocyclic or heterocyclic group, wherein R₂ is selected from H, halogen, -OH, and -O(C1-10)alkyl.

11. A compound according to any preceding claim, wherein Rₐ represents H, (C1-10)alkyl, -OR₅, halogen, -NO₂, or a polar moiety, wherein R₅ represents H, (C1-10)alkyl, -Phe, or -Bn.

12. A compound according to any preceding claim, wherein R_{b} represents H, (C1-10) alkyl, -OR₇, -COOR₇, -CONR₇R₈, halogen, - CF₃, or a polar moiety, wherein R₇ and R₈ represent H or (C1-10)alkyl.

13. A compound according to any preceding claim, wherein the polar moiety is selected from glucoronides, polyhydroxy groups, 2,3-dihydroxy propoxy groups, tartrate groups, N-(2-Hydroxyethyl)-iminodiacetic acid groups, tetrazolium salt groups, phosphate groups, sulfonate groups, sulfonic acid groups, aminoalkyl groups, alkylammonium groups, quaternary ammonium salts and derivatives thereof.

14. A compound according to any preceding claim, wherein the polar moiety is selected from polyhydroxy groups, tartrate groups, N-(2-Hydroxyethyl)-iminodiacetic acid groups, phosphate groups, sulfonate groups, aminoalkyl groups, alkylammonium groups, and derivatives thereof.

15. A compound according to any preceding claim, wherein n is 1.

16. A compound according to any preceding claim, wherein m is 1.

17. A compound according to any preceding claim, wherein n and m are 1.

18. A compound according to claim 1 having the formula II or a pharmaceutically acceptable salt or solvate thereof, 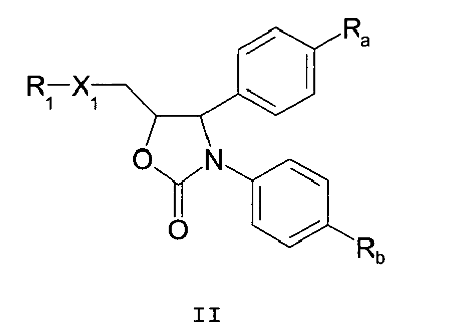 wherein
X₁ is -O-, -NH-, -N=, -NH-CO-, -O-CO-NH-, -NH-CO-NH-, or combinations thereof,
R₁ is H or R₂-substituted aryl Ar, wherein R₂ represents H, -OH, -O(C1-10)alkyl, -CF₃, -CN, or halogen;
Rₐ represents H, (C1-10)alkyl, -OR₅, halogen, -NO₂, or a polar moiety, wherein R₅ represents H, (C1-10)alkyl, -Phe, or -Bn;
R_{b} represents H, (C1-10)alkyl, -OR₇, -COOR₇, -CONR₇R₈, halogen, - CF₃, or a polar moiety, wherein R₇ and R₈ represent H or (C1-10)alkyl.

19. A compound according to claim 18, wherein when Rₐ and R_{b} equal -OBn and -F, respectively -X₁-R₁ may not be -OH

20. A compound according to claim 1, having the formula III or a pharmaceutically acceptable salt or solvate thereof, 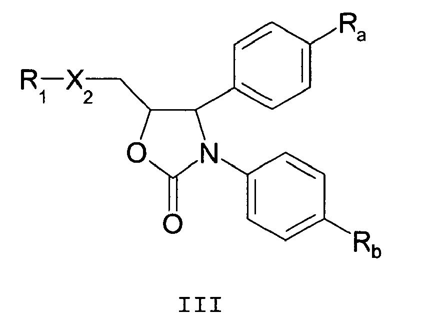 wherein
X₂ is a five- or six-membered ring having 1 to 3 heteroatoms selected from N, O, S and benzo-fused derivatives thereof;
R₁ is H or R₂-substituted aryl Ar, wherein R₂ represents H, -OH, -O(C1-10)alkyl, -CF₃, -CN, or halogen; and
Rₐ represents H, (C1-10)alkyl, -OR₅, halogen, -NO₂, or a polar moiety, wherein R₅ represents H, (C1-10)alkyl, -Phe, or -Bn;
R_{b} represents H, (C1-10)alkyl, -OR₇, -COOR₇, -CONR₇R₈, halogen, - CF₃, or a polar moiety, wherein R₇ and R₈ represent H or (C1-10)alkyl.

21. A compound according to claim 20, wherein X₂ is selected from five- or six-membered rings having 1 to 3 heteroatoms selected from N, O, S and benzo-fused derivatives thereof.

22. A compound according to claims 20 or 21, wherein X₂ is selected from pyridine, pyrimidine, oxazole, thiazole, imidazole, triazole, pyrazole and phtaleimide.

23. A compound according to any of claims 20 to 22, wherein R₂-substituted aryl Ar is a five- or six-membered aromatic, carbocyclic or heterocyclic group, preferably phenyl, pyridyl, naphthyl, wherein R₂ is selected from H, halogen, -OH, and - O(C1-10)alkyl.

24. A compound according to any of claims 18 to 23, wherein the polar moiety is selected from cyano, glucoronide, polyhydroxy groups, 2,3-dihydroxy propoxy groups, tartrate groups, N-(2-Hydroxyethyl)-iminodiacetic acid groups, tetrazolium salt groups, phosphate groups, sulfonate groups, sulfonic acid groups, aminoalkyl groups, alkylammonium groups, quaternary ammonium salts and derivatives thereof.

25. A compound according to any preceding claim, wherein the substituents in the 4- and 5-position are in trans configuration to each other.

26. A pharmaceutical composition comprising a therapeutically effective amount of a compound of any preceding claim with a pharmaceutically acceptable carrier.

27. A pharmaceutical composition according to claim 26 further comprising one or more additional active agents.

28. A pharmaceutical composition according to claims 26 or 27 for the treatment or prevention of artheriosclerosis or for the reduction of cholesterol levels.

29. A kit comprising a compound according to claims 1 to 25 or a pharmaceutical composition according to claims 26 or 27 for use in the treatment or prevention of artheriosclerosis or for the reduction of cholesterol levels.

30. A method for the treatment or prevention of artheriosclerosis or for the reduction of cholesterol levels comprising administering to a subject in need of such treatment an effective amount of a compound according to claims 1 to 25 or a pharmaceutical composition according to claims 26 or 27.

31. Use of a compound according to claims 1 to 25 for the manufacture of a medicament for the treatment or prevention of artheriosclerosis or for the reduction of cholesterol levels.
